# EUROPEAN PATENT SPECIFICATION

(11) **EP 3 431 988 B1**
(45) Date of publication and mention of the grant of the patent: **10.03.2021**
(21) Application number: 17181908.9
(22) Date of filing: 18.07.2017
(51) Int. Cl.: G01N 33/50, G01N 33/569

(54) **METHOD TO DETERMINE THE STATE OF THE HUMORAL IMMUNE SYSTEM IN A PATIENT**
VERFAHREN ZUR BESTIMMUNG DES ZUSTANDS DES HUMORALEN IMMUNSYSTEMS BEI EINEM PATIENTEN
PROCÉDÉ POUR DÉTERMINER L'ÉTAT DU SYSTÈME IMMUNITAIRE HUMORAL CHEZ UN PATIENT

(43) Date of publication of application: 23.01.2019
(73) Proprietor: CBmed GmbH Center for Biomarker Research in Medicine, 8010 Graz (AT)
(72) Inventor: HORVATH, Angela, 8010 Graz (AT); STADLBAUER-KÖLLNER, Vanessa, 8010 Graz (AT); LEBER, Bettina, 8010 Graz (AT); SCHMERBÖCK, Bianca, 8010 Graz (AT); RAINER, Florian, 8010 Graz (AT)
(74) Representative: Geling, Andrea

(56) References cited:
- WO-A1-01/81429
- WO-A1-2016/139092
- WO-A2-2009/108170
- US-A1- 2011 097 344
- LAMONTAGE A. ET AL.: "Altered functionality of anti-bacterial antibodies in patients with chronic hepatitis C virus infection", PLOS ONE, vol. 8, no. 6, E64992, June 2013 (2013-06) , pages 1-11, XP002773717, DOI: 10.1371/journal.pone.0064992

## Description

The present invention relates to method of determining the state of the humoral immune system in a patient. The present invention further relates to a method of determining the susceptibility of a patient to develop an infection. The present invention also relates to a method of monitoring the state of the humoral immune system in a patient. In addition, the present invention relates to a method of monitoring the susceptibility of a patient to develop an infection. Moreover, the present invention relates to the use of a kit for determining the state of the humoral immune system in a patient, the susceptibility of a patient to develop an infection, monitoring the state of the humoral immune system in a patient, and/or monitoring the susceptibility of a patient to develop an infection.

### BACKGROUND OF THE INVENTION

The immune system is the host's tool to fight pathogens causing infections and other foreign material that gets into the host's body. The host's defense system comprises many biological structures and processes that protect against diseases. To function properly, an immune system must detect a wide variety of agents, known as pathogens, from viruses to parasitic worms, and distinguish them from the organism's own healthy tissue.

The skin, liver, spleen, kidneys, bloodstream, and lymphatic system are most important in protecting host's immunity to these foreign agents. Impairment of any of these organs (for example, liver infection or failure, poor skin quality and predisposition to wounds/sores) will result in an impaired or weaker immune system. Poor nutrition and poor overall health status are significant contributors to a weakened immune system.

In many species, the immune system can be classified into subsystems, such as the innate immune system versus the adaptive immune system, or humoral immunity versus cell-mediated immunity.

An impaired immune system may be acquired through disease (e.g. liver disease) or medication (e.g. steroids, chemotherapy, or transplant medication to prevent rejection). The immune system can also turn against the host (auto-immune diseases) and cause illnesses.

The ability of the host's body to kill or inhibit the growth of pathogens such as bacteria in the blood or tissue has long been of interest in different diseases such as liver diseases. For example, dysfunctional or downregulated complement factors have been described in alcoholic cirrhotic (Homann C. et al. "Acquired C3 deficiency in patients with alcoholic cirrhosis predisposes to infection and increased mortality", Gut, 1997, 40, pages 544 to 549) and the downregulation of antimicrobial peptides (AMPs) in the intestine has been recently shown to play a pivotal role in the genesis of alcoholic liver disease (Wang L. et al. "Intestinal REG3 Lectins protect against alcoholic steatohepatitis by reducing mucosa-associated microbiota and preventing bacterial translocation", Cell Host Microbe, 2016, 19, pages 227 to 239). The immune system plays a dual role in the pathogenesis of liver diseases such as cirrhosis such that, besides the role of immune-mediated inflammatory mechanisms, cirrhosis itself also leads to immune system dysfunction. The immune system mediates hepatocyte damage due to alcohol, virus infection or autoimmunity, driving fibrogenesis through hepatic stellate cell activation. In addition, cirrhosis leads to impairment of the immune system with an inability to protect the host from bacterial infection and dysregulated immune cell activation.

To measure the response of an individual's immune system is of interest in order to determine the individual's immune status. Knowing the status of individual's immune system allows, for example, treatment as well as living habit adaptations.

Only a few protocols are published so far that allow the examination of the killing capacity of blood of an individual. Said protocols are not easy to handle, time- and work-intensive, and/or unreliable. They involve, for example, the addition of complement factors (Lamontage A. et al. "Altered functionality of anti-bacterial antibodies in patients with chronic hepatitis C virus infection", PLoS One, 2013, 8, e64992). Thus, there is a need for the establishment of an assay to determine the status of the immune system in an individual which is easy in use, suitable for clinical routine or medical practice assessment and reliable.

The present inventors have established an assay which utilizes the ability of individual's blood to kill bacteria as a read out to determine the status of the humoral immune system of said individual. Said assay fulfils the above mentioned requirements and, thus, can be used on a routinely basis in clinics and doctor's offices.

The present inventors have further established an assay which utilizes the ability of individual's blood to kill bacteria as a read out to determine the susceptibility of an individual to develop an infection.

### SUMMARY OF THE INVENTION

In a first aspect, the present invention relates to a method of determining the state of the humoral immune system in a patient comprising the steps of:
(i) providing a mixture consisting of a blood sample obtained from the patient and a bacteria suspension, wherein the bacteria are antimicrobial peptide (AMP) sensitive,
(ii) incubating the mixture, and
(iii) determining the change in density of the mixture over time.

In a second aspect, the present invention relates to a method of determining the susceptibility of a patient to (develop) an infection comprising the steps of:
(i) providing a mixture consisting of a blood sample obtained from the patient and a bacteria suspension, wherein the bacteria are antimicrobial peptide (AMP) sensitive,
(ii) incubating the mixture, and
(iii) determining the change in density of the mixture over time.

In a third aspect, the present invention relates to a method of monitoring the state of the humoral immune system in a patient comprising the steps of:
(a) carrying out an incubation cycle comprising
   (i) providing a mixture consisting of a blood sample obtained from the patient and a bacteria suspension, wherein the bacteria are antimicrobial peptide (AMP) sensitive,
   (ii) incubating the mixture, and
   (iii) determining the change in density of the mixture over time, wherein said determining comprises determining the density of the mixture at least at one first point in time and at least at one second point in time and comparing the densities determined at the different time points, and
(b) carrying out at least one further incubation cycle comprising
   (i) providing a mixture consisting of a blood sample obtained from the patient and a bacteria suspension, wherein the bacteria are antimicrobial peptide (AMP) sensitive,
   (ii) incubating the mixture, and
   (iii) determining the change in density of the mixture over time, wherein said determining comprises determining the density of the mixture at least at one third point in time and at least at one fourth point in time and comparing the densities determined at the different time points.

In a fourth aspect, the present invention relates to a method of monitoring the susceptibility of a patient to (develop) an infection comprising the steps of:
(a) carrying out an incubation cycle comprising
   (i) providing a (first) mixture consisting of a blood sample obtained from the patient and a bacteria suspension, wherein the bacteria are antimicrobial peptide (AMP) sensitive,
   (ii) incubating the (first) mixture, and
   (iii) determining the change in density of the (first) mixture over time, wherein said determining comprises determining the density of the (first) mixture at least at one first point in time and at least at one second point in time and comparing the densities determined at the different time points, and
(b) carrying out at least one further incubation cycle comprising
   (i) providing a (second) mixture consisting of a blood sample obtained from the (same) patient and a bacteria suspension, wherein the bacteria are antimicrobial peptide (AMP) sensitive,
   (ii) incubating the (second) mixture, and
   (iii) determining the change in density of the (second) mixture over time, wherein said determining comprises determining the density of the (second) mixture at least at one third point in time and at least at one fourth point in time and comparing the densities determined at the different time points.

In a fifth aspect, the present invention relates to the use of a bacteria suspension to determine the state of the humoral immune system in a blood sample obtained from a patient, wherein the bacteria suspension and the blood sample form a mixture consisting of the bacteria suspension and the blood sample, and wherein the bacteria are antimicrobial peptide (AMP) sensitive.

In a sixth aspect, the present invention relates to the use of a bacteria suspension to determine the susceptibility of a patient to develop an infection in a blood sample obtained from the patient, wherein the bacteria suspension and the blood sample form a mixture consisting of the bacteria suspension and the blood sample, and wherein the bacteria are antimicrobial peptide (AMP) sensitive.

In a seventh aspect, the present invention relates to the use of a bacteria suspension to monitor the state of the humoral immune system in a blood sample obtained from a patient, wherein the bacteria suspension and the blood sample form a mixture consisting of the bacteria suspension and the blood sample, and wherein the bacteria are antimicrobial peptide (AMP) sensitive.

In an eight aspect, the present invention relates to the use of a bacteria suspension to monitor the susceptibility of a patient to develop an infection in a blood sample obtained from the patient, wherein the bacteria suspension and the blood sample form a mixture consisting of the bacteria suspension and the blood sample, and wherein the bacteria are antimicrobial peptide (AMP) sensitive.

In a ninth aspect, the present invention relates to the use of a kit for determining the state of the humoral immune system in a patient, the susceptibility of a patient to develop an infection, monitoring the state of the humoral immune system in a patient, and/or monitoring the susceptibility of a patient to develop an infection consisting of: (i) antimicrobial peptide (AMP) sensitive bacteria, (ii) means for sampling blood from a patient, and/or (iii) means for preparing a suspension of said bacteria and/or cultivating the suspension of said bacteria and/or a mixture consisting of the suspension of said bacteria and the blood sample.

This summary of the invention does not necessarily describe all features of the present invention. Other embodiments will become apparent from a review of the ensuing detailed description.

### DETAILED DESCRIPTION OF THE INVENTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kolbl, H. eds. (1995), Helvetica Chimica Acta, CH-4010 Basel, Switzerland).

Several documents are cited throughout the text of this specification. Nothing herein is to be construed as an admission that the invention is not entitled to antedate such disclosure by virtue of prior invention. In the event of a conflict between the definitions or teachings of such incorporated references and definitions or teachings recited in the present specification, the text of the present specification takes precedence.

The term "comprise" or variations such as "comprises" or "comprising" according to the present invention means the inclusion of a stated integer or group of integers but not the exclusion of any other integer or group of integers. The term "consisting essentially of' according to the present invention means the inclusion of a stated integer or group of integers, while excluding modifications or other integers which would materially affect or alter the stated integer. The term "consisting of' or variations such as "consists of' according to the present invention means the inclusion of a stated integer or group of integers and the exclusion of any other integer or group of integers.

The terms "a" and "an" and "the" and similar reference used in the context of describing the invention (especially in the context of the claims) are to be construed to cover both the singular and the plural, unless otherwise indicated herein or clearly contradicted by context.

The term "immune system", as used herein, refers to a host defense system comprising many biological structures and processes within an organism that protects against diseases. To function properly, an immune system must detect a wide variety of agents, known as pathogens, from viruses to parasitic worms (e.g. bacteria), and distinguish them from the organism's own healthy tissue. In many species, the immune system can be classified into subsystems, such as the innate immune system versus the adaptive immune system, or humoral immunity versus cell-mediated immunity.

The term "immune response", as used herein, relates to a reaction of the immune system to immunogenic organisms, such as bacteria, viruses, cells or other substances. The term "immune response" includes the innate immune response and the adaptive immune response. In particular, the immune response is related to an activation of immune cells, an induction of cytokine biosynthesis, and/or antibody production.

The term "innate immune system" (also known as "non-specific immune system" or "in-born immune system"), as used herein, refers to a subsystem of the overall immune system that comprises the cells and mechanisms that defend the host from infection by other organisms. The cells of the innate system recognize and respond to pathogens such as bacteria in a generic way, but, unlike the adaptive immune system, the system does not provide long-lasting immunity to the host. Innate immune systems provide immediate defense against infections such as bacterial infections. Like the adaptive system, the innate system includes both humoral immunity components and cell-mediated immunity components.

The term "humoral immune system", as used herein, refers to the aspect of immunity that is mediated by macromolecules found in extracellular fluids such as secreted antibodies, complement proteins, and antimicrobial peptides (AMPs). It is so named because it involves substances found in the humors, or body fluids such as blood (e.g. serum or plasma). Humoral immunity also refers to antibody production and the accessory processes that accompany it, including: Th2 activation and cytokine production, germinal center formation and isotype switching, affinity maturation and memory cell generation. It also refers to the effector functions of antibodies, which include pathogen such as bacteria and toxin neutralization, classical complement activation, and opsonin promotion of phagocytosis and pathogen elimination. It further refers to AMP production and pathogen (e.g. bacteria) neutralization as well as pathogen (e.g. bacteria) elimination/killing by AMPs. Instead of the term "humoral immune system", the term "humoral immunity" may be used.

The term "antimicrobial peptides (AMPs)" (also called "host defence peptides (HDPs)"), as used herein, refers to molecules which are part of the innate immune response and offer protection from invading pathogens. AMPs show potent antimicrobial activity against bacteria such as gram-positive and gram-negative bacteria, fungi, parasites and viruses. They have generally a length of between 12 and 50 amino acids. AMPs act by disrupting the structure or function of microbial cell membranes. They do not target single defined molecular structures. Therefore, as opposed to conventional antibiotics, they are effective regardless of the metabolic activity of bacteria. Human AMPs such as defensins and cathelicidin (LL-37) are present in leukocytes and secreted by various epithelia in skin and mucosal surfaces. In addition to their antimicrobial activity, AMPs are important effector molecules in inflammation, immune activation, and wound healing. AMPs are quite diverse in sequence and secondary structure, but share some common properties. They are usually cationic, amphipathic and exert their microbicidal effect by compromising the bacterial membrane integrity. Interaction of AMPs with the anionic membrane surface of the target microbes leads to membrane permeabilization, cell lysis and death. It is generally accepted that the cytoplasmic membrane is the main target of most AMPs, whereby accumulation of peptide in the membrane causes increased permeability and loss of barrier function resulting in leakage of cytoplasmic components and cell death.

The term "antimicrobial peptide (AMP) sensitive bacteria", as used herein, refers to bacteria which can be targeted and eliminated/killed by AMPs. Said AMP sensitive bacteria may be gram-positive and gram-negative bacteria. Said AMP sensitive bacteria may also be aerobic bacteria. In a preferred embodiment, said AMP sensitive bacteria are gram-negative and aerobic bacteria. In a more preferred embodiment, the AMP sensitive bacteria are *E.coli* bacteria, in particular *E.coli* XL1 blue bacteria.

The term "determination of the status of the humoral immune system in a patient", as used herein, refers to the evaluation of the condition of the humoral immune system in a patient. It may be determined that the patient has an intact, unchanged, or improved humoral immune system. It may also be determined that the patient has a deteriorated, in particular an impaired, humoral immune system. Unchanged or improved in this respect preferably means unchanged or improved compared to a control/reference. The control/reference may be the same patient (measurements are made at different points in time which are then compared with each other) or a different control/reference subject (measurements are compared to the values achieved for the control/reference subject).

The term "intact humoral immune system", as used herein, refers to an immune system which is able to respond to and eliminate infectious agents/pathogens, such as bacteria, viruses, and fungi, from the patient's body. The elimination of infectious agents normally takes place by macromolecules such as secreted antibodies, complement proteins, and antimicrobial peptides (AMPs) which neutralize and/or disrupt said infectious agents/pathogens.

The term "deteriorated humoral immune system", as used herein, refers to an immune system which responsiveness to infectious agents such as bacteria, viruses, and fungi and its ability to eliminate said infectious agents from the patient's body is weakened. As mentioned above, the elimination of infectious agents normally takes place by macromolecules such as secreted antibodies, complement proteins, and certain antimicrobial peptides (AMPs) which neutralize and/or disrupt said infectious agents/pathogens.

The term "impaired humoral immune system", as used herein, refers to an immune system which responsiveness to infectious agents such as bacteria, viruses, and fungi and its ability to eliminate said infectious agents from the patient's body is hindered or disrupted. As mentioned above, the elimination of infectious agents normally takes place by macromolecules such as secreted antibodies, complement proteins, and certain antimicrobial peptides (AMPs) which neutralize and/or disrupt said infectious agents/pathogens. An unchecked infection leads to sepsis which can result in the death of the patient.

A deteriorated, in particular an impaired, humoral immune system is frequently observed as a secondary effect of conditions such as trauma, for example, from an accident or from undergoing a major surgical procedure, from a disease such as debilitating disease, e.g. cancer or an infection, or from malnutrition or old age. The disease may be a liver disease, e.g. liver cirrhosis, liver fibrosis, hepatic steatosis, or hepatitis virus infection such as hepatitis C virus infection like chronic hepatitis C virus infection. The infection may be an urinary tract infection, a skin and soft tissue infection, an airway infection, a spontaneous bacterial peritonitis, a sepsis, and a gastrointestinal infection.

In the context of the present invention, the ability of the patient's humoral immune system to respond to AMP sensitive bacteria is tested. In particular, the ability of AMPs, comprised in a blood sample obtained from a patient, to kill/eliminate bacteria which have been added to the blood sample is determined according to the present invention in order to evaluate the status of the humoral immune system in the patient. The patient may be determined as having an intact or a deteriorated, in particular an impaired, humoral immune system.

The term "determination of the susceptibility of a patient to (develop) an infection", as used herein, refers to the evaluation of a patient's sensitivity or vulnerability to (develop) an infection. It may be determined that the patient has no susceptibility to (develop) an infection. It may also be determined that the patient has a susceptibility to (develop) an infection.
A patient having a deteriorated, in particular an impaired, humoral immune system may be susceptible to (develop) an infection. The patient may have an increased risk of infectious complications because of a deficiency in any of his (or her) defense mechanisms, (e.g., skin barrier, mucous membrane barrier, innate immune, humoral immune, or cellular immunity).
In addition, a patient having an intact humoral immune system may be not susceptible to (develop) an infection. A subject having an intact humoral immune system and without a known deficiency in any defense mechanism is referred to herein as being healthy. Such a patient may also be designated as immune competent. It may, however, suffer from another disease independent from or not related to the immune system.

The term "patient", as used herein, refers to any subject for whom the state of the humoral immune system is desired to be known. It may be determined that the patient has an intact or an impaired humoral immune system. It may also be determined that the patient has a deteriorated or an improved humoral immune system. It may further be determined that the patient has an unchanged humoral immune system.
The term "patient", as used herein, also refers to a subject which has an impaired humoral immune system. The status of the humoral immune system of the patient may be retested and it may be determined that the patient has still an impaired humoral immune system or not an impaired humoral immune system anymore, e.g. after therapeutic intervention. The status of the humoral immune system of the patient may also be retested and it may be determined that the patient has still a deteriorated humoral immune system or not a deteriorated humoral immune system anymore, e.g. after therapeutic intervention. In addition, the status of the humoral immune system of the patient may be retested and it may be determined that the status of the humoral immune system of the patient has worsened, improved, or is unchanged.
The patient mentioned above may be a patient suffering from a disease, in particular from a disease affecting the humoral immune system of the patient, e.g. a liver disease.
The patient as mentioned above may be a patient which has undergone or undergoes a treatment of a disease, in particular a disease affecting the humoral immune system of the patient, e.g. a liver disease.
It should be noted that a patient who has an intact or improved humoral immune system may possibly suffer from a disease or condition not tested/known, in particular from a disease or condition not affecting the humoral immune system.

Alternatively, the term "patient", as used herein, refers to any subject for whom the susceptibility to (develop) an infection is desired to be known. It may be determined that the patient has a susceptibility to (develop) an infection or has no susceptibility to (develop) an infection.
The term "patient", as used herein, also refers to a subject which has a susceptibility to develop an infection. The status of the humoral immune system of the patient may be retested and it may be determined that the patient has still a susceptibility to (develop) an infection or no susceptibility to (develop) an infection anymore, e.g. after therapeutic intervention.
The patient having no susceptibility to develop an infection may be a patient having an intact or improved humoral immune system. The patient having a susceptibility to develop an infection may be a patient having a deteriorated, in particular an impaired, humoral immune system.
The patient mentioned above may be a patient suffering from a disease, e.g. a liver disease. The liver disease may be selected from the group consisting of liver cirrhosis, liver fibrosis, hepatic steatosis, and hepatitis virus infection such as hepatitis C virus infection like chronic hepatitis C virus infection. In particular, the patient may be a cirrhotic patient with alcohol as aetiological entity, a cirrhotic patient with a (chronic) hepatitis C infection as aetiological entity, a cirrhotic patient with a variety of causing factors including, but not limited to, non-alcoholic fatty liver disease (NALFD), hemochromatosis, primary biliary cirrhosis, (chronic) hepatitis B infection, primary sclerosing cholangitis, Morbus Wilson and/or unclear reasons, a patient with a (chronic) hepatitis C infection, a patient with a (chronic) hepatitis C infection and a liver fibrosis, or a patient with a liver fibrosis.
The patient as mentioned above may be a patient which has undergone or undergoes a treatment of a disease, e.g. a liver disease.
It should be noted that a patient who has no susceptibility to develop an infection may possibly suffer from another disease or condition not tested/known.

The patient may be any mammal, including both a human and another mammal, e.g. an animal such as a rabbit, mouse, rat, or monkey. Human patients are particularly preferred.

The term "(control) subject", as used herein, refers to a subject known to have an intact humoral immune system or a deteriorated, in particular an impaired, humoral immune system. It should be noted that a (control) subject having an intact humoral immune system may possibly suffer from a disease or condition not tested/known, in particular from a disease or condition not affecting the humoral immune system.

Alternatively, the term "(control) subject", as used herein, refers to a subject known to have no susceptibility to (develop) an infection. Said (control) subject may have an intact humoral immune system. The "(control) subject", as used herein, also refers to a subject known to have a susceptibility to (develop) an infection. Said (control) subject may have a deteriorated, in particular an impaired, humoral immune system.
It should be noted that a (control) subject having no susceptibility to develop an infection may possibly suffer from a disease or condition not tested/known.

The (control) subject may be any mammal, including both a human and another mammal, e.g. an animal such as a rabbit, mouse, rat, or monkey. Human (control) subjects are particularly preferred.

The term "treatment", in particular "therapeutic treatment", as used herein, refers to any therapy which improves the health status and/or prolongs (increases) the lifespan of a patient. Said therapy may eliminate the disease in a patient, arrest or slow the development of a disease in a patient, inhibit or slow the development of a disease in a patient, decrease the frequency or severity of symptoms in a patient, and/or decrease the recurrence in a patient who currently has or who previously has had a disease. The treatment of the disease may be selected from the group consisting of drug administration, nutrition adaptation, chemotherapy, surgery, and radiotherapy. In a preferred embodiment, the patient suffers from a liver disease. The treatment of a liver disease may be selected from the group consisting of drug administration, surgery, nutritional intervention, neutralization of aetiological entities, abstinence from hepatotoxic substances (e.g. alcohol), and therapeutic phlebotomy. Preferably, the liver disease is selected from the group consisting of liver cirrhosis, liver fibrosis, hepatic steatosis, and hepatitis virus infection, more preferably hepatitis C virus infection, even more preferably chronic hepatitis C virus infection. In particular, the therapeutic treatment may be an antiviral treatment, specifically in patients suffering from a (chronic) hepatitis C virus infection. The state of the humoral immune system of the patient suffering from a (chronic) hepatitis C virus infection usually improves after antiviral therapy. Once the virus is reduced/eradicated, a significant improvement can be observed/determined.

The term "disease", as used herein, refers to an abnormal condition that affects the body of an individual. A disease is often construed as a medical condition associated with specific symptoms and signs. A disease may be caused by factors originally from an external source, such as infectious disease, or it may be caused by internal dysfunctions, such as autoimmune diseases. In humans, "disease" is often used more broadly to refer to any condition that causes pain, dysfunction, distress, social problems, or death to the individual afflicted, or similar problems for those in contact with the individual. In this broader sense, it sometimes includes injuries, disabilities, disorders, syndromes, infections, isolated symptoms, deviant behaviors, and atypical variations of structure and function, while in other contexts and for other purposes these may be considered distinguishable categories. Diseases usually affect individuals not only physically, but also emotionally, as contracting and living with many diseases can alter one's perspective on life, and one's personality.
In a preferred embodiment, the patient suffers from a liver disease. Preferably, the liver disease is selected from the group consisting of liver cirrhosis, liver fibrosis, hepatic steatosis, and hepatitis virus infection, more preferably hepatitis C virus infection, even more preferably chronic hepatitis C virus infection.

The term "infectious disease" (or simply "infection"), as used herein, refers to any disease which can be transmitted from individual to individual or from organism to organism, and is caused by a microbial agent. Infectious diseases are known in the art and include, for example, a viral disease, a bacterial disease, or a parasitic disease. Said diseases are caused by a virus, a bacterium, and a parasite, respectively. In this regard, the infectious disease can be, for example, hepatitis, sexually transmitted diseases (e.g. chlamydia or gonorrhea), tuberculosis, HIV/acquired immune deficiency syndrome (AIDS), diphtheria, hepatitis B, hepatitis C, cholera, severe acute respiratory syndrome (SARS), the bird flu, and influenza. In a preferred embodiment, the infectious disease/infection is selected from the group consisting of an urinary tract infection, a skin and soft tissue infection, an airway infection, a spontaneous bacterial peritonitis, a sepsis, and a gastrointestinal infection.

The term "blood sample", as used herein, encompasses whole blood or a blood fraction such as serum, plasma, or blood cells. Serum and plasma represent the extracellular component of whole blood (blood extracellular fraction). In contrast thereto, the blood cells represent the cellular component of whole blood (blood cellular fraction). They include erythrocytes, leukocytes and thrombocytes. The blood cell sample may comprise or consist of erythrocytes, leukocytes, and/or thrombocytes. The blood sample may have a volume of between 0.1 and 20 ml, more preferably of between 0.5 and 10 ml, i.e. 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, or 20 ml. Preferably, the blood sample is serum or plasma. With respect to plasma, it is particularly preferred that the plasma is not EDTA-plasma. The plasma may be, however, lithium heparin-plasma or sodium citrate-plasma.

The term "bacteria suspension" (also designated as "bacterial suspension"), as used herein, refers to a formulation comprising bacteria and culture medium, a buffer solution, or water. The culture medium preferably comprises nutrients and minerals which support the growth of the bacteria. Thus, the culture medium may also be designated as growth medium. In cases where the bacteria are cultured alone, i.e. without the addition of a blood sample obtained from a patient, the bacteria suspension comprises bacteria and a culture (growth) medium. The bacteria suspension is cultured alone for reference cases. It is, thus, also designated as reference bacteria suspension. In cases described herein where the bacteria suspension is added to a blood sample or the blood sample is added to a bacteria suspension in order to generate a mixture of the blood sample and bacteria suspension, the bacteria suspension comprises bacteria and a culture medium, buffer solution, or water. Preferably, the bacteria suspension added to the blood sample or to which the blood sample is added has an optical density at 600 nm of between 0.1 and 0.2, more preferably of between 0.12 and 0.18, and even more preferably of between 0.15 and 0.18. Preferably, the mixture of the bacteria suspension and blood sample has (immediately) after its preparation and/or at the beginning of the incubation phase/period has an optical density at 600 nm of between 0.1 and 0.3, more preferably of between 0.1 and 0.15, and even more preferably of between 0.12 and 0.15. Preferably, the bacteria suspension cultured alone has at the beginning of the incubation phase/period an optical density at 600 nm of between 0.1 and 0.13, more preferably of between 0.1 and 0.12, and even more preferably of between 0.11 and 0.12. In the context of the present invention, the bacteria are AMP sensitive bacteria.

It is known in the field of microbiology that the turbidity of a bacteria suspension is a measure of bacteria density. The term "density", as used herein, refers to the optical density (OD) of the bacteria or the number of bacteria per volume unit. While the number of bacteria per volume unit may be determined by counting (e.g. using a counting chamber), the optical density may be determined by light scattering techniques, e.g. via the McFahrland Turbidity Standards or using a spectrophotometer.
The McFarland standards are used as a reference to adjust the turbidity of bacterial suspensions so that the number of bacteria will be within a given range to standardize microbial testing. An example of such testing is antibiotic susceptibility testing by measurement of minimum inhibitory concentration which is routinely used in medical microbiology and research. If a suspension used is too heavy or too dilute, an erroneous result (either falsely resistant or falsely susceptible) for any given anti-microbial agent could occur. Original McFarland standards were mixing specified amounts of barium chloride and sulfuric acid together. Mixing the two compounds forms a barium sulfate precipitate, which causes turbidity in the solution. A 0.5 McFarland standard is prepared by mixing 0.05 mL of 1.175% barium chloride dihydrate (BaCl₂•2H₂O), with 9.95 mL of 1% sulfuric acid (H₂SO₄). Now there are McFarland standards prepared from suspensions of latex particles, which lengthens the shelf life and stability of the suspensions. The standard can be compared visually to a suspension of bacteria in sterile saline or nutrient broth. If the bacterial suspension is too turbid, it can be diluted with more diluent. If the suspension is not turbid enough, more bacteria can be added.
The spectrophotometer method measures turbidity directly. The best case (i.e. most sensitive) would be to have a narrow slit and a small detector so that only the light scattered in the forward direction would be seen by the detector. This instrument would give larger apparent absorption readings than other instruments.
As should be obvious, each spectrophotometer used must be independently calibrated for use in estimating microbial concentrations. Not only is the apparent absorption affected by the width of the instrument's slit, the condition of the filter, and the size and condition of the detector, but also each time the lamp is changed the calibration needs to be repeated as different bulbs may vary in total output.
The optical density is preferably measured at a wave length of between 400 and 800 nm, more preferably at a wave length of 600 nm.

The term "bacteria growth", as used herein, refers to the increase of the number of bacteria/bacterial cells over time. The growth of bacteria in batch culture can be modeled with four different phases: lag phase, log phase or exponential phase, stationary phase, and death phase. During the lag phase, bacteria adapt themselves to growth conditions. It is the period where the individual bacteria are maturing and not yet able to divide. During the lag phase of the bacterial growth cycle, synthesis of RNA, enzymes and other molecules occurs. The log phase (sometimes called the logarithmic phase or the *exponential phase*) is a period characterized by cell doubling. The number of new bacteria appearing per unit time is proportional to the present population. If growth is not limited, doubling will continue at a constant rate so both the number of cells and the rate of population increase doubles with each consecutive time period. For this type of exponential growth, plotting the natural logarithm of cell number against time produces a straight (linear) line. The slope of this line is the specific growth rate of the organism, which is a measure of the number of divisions per cell per unit time. The actual rate of this growth (i.e. the slope of the line in the figure) depends upon the growth conditions, which affect the frequency of cell division events and the probability of both daughter cells surviving. Exponential growth cannot continue indefinitely, however, because the medium is soon depleted of nutrients and enriched with wastes. The stationary phase is often due to a growth-limiting factor such as the depletion of an essential nutrient, and/or the formation of an inhibitory product such as an organic acid. Stationary phase results from a situation in which growth rate and death rate are equal. The number of new cells created is limited by the growth factor and as a result the rate of cell growth matches the rate of cell death. The result is a "smooth," horizontal linear part of the curve during the stationary phase. At death phase (decline phase), bacteria die. This could be caused by lack of nutrients, environmental temperature above or below the tolerance band for the species, or other injurious conditions. The bacteria incubated in the methods of the present invention are preferably cultured in a batch culture.
The methods described herein are preferably carried out during the log phase of the bacteria. In particular, the bacteria comprised in the mixture or suspension are grown to log phase. In other words, the bacteria comprised in the mixture or suspension show particularly an exponential growth. This exponential growth appears as a linear increase in optical density or extinction.

In the context of the present invention, the term "kit of parts (in short: kit)" is understood to be any combination of at least some of the components identified herein, which are combined, coexisting spatially, to a functional unit, and which can contain further components. Said kit may allow point-of-care testing (POCT).

The term "point-of-care testing (POCT)", as used herein, refers to a medical diagnostic testing at or near the point of care that is the time and place of patient care. This contrasts with the historical pattern in which testing was wholly or mostly confined to the medical laboratory, which entailed sending off specimens away from the point of care and then waiting hours or days to learn the results, during which time care must continue without the desired information. Point-of-care tests are simple medical tests that can be performed at the bedside. The driving notion behind POCT is to bring the test conveniently and immediately to the patient. This increases the likelihood that the patient, physician, and care team will receive the results quicker, which allows for immediate clinical management decisions to be made. POCT is often accomplished through the use of transportable, portable, and handheld instruments and test kits. Small bench analyzers or fixed equipment can also be used when a handheld device is not available - the goal is to collect the specimen and obtain the results in a very short period of time at or near the location of the patient so that the treatment plan can be adjusted as necessary before the patient leaves.

### Embodiments of the invention

As mentioned above, only a few protocols are published so far that allow the examination of the killing capacity of blood of an individual. Said protocols are not easy to handle, time- and work-intensive, and/or unreliable. They involve, for example, the addition of complement factors (Lamontage A. et al. "Altered functionality of anti-bacterial antibodies in patients with chronic hepatitis C virus infection", PLoS One, 2013, 8, e64992). Thus, there is a need for the establishment of an assay to determine the status of the immune system in an individual which is easy in use, suitable for clinical routine or medical practice assessment and reliable.

The present inventors have established an assay which utilizes the ability of individual's blood to kill bacteria as a read out to determine the status of the humoral immune system of said individual. Said assay fulfils the above mentioned requirements and, thus, can be used on a routinely basis in clinics and doctor's offices.

Thus, in a first aspect, the present invention relates to a method of determining the state of the humoral immune system in a patient comprising the steps of:
(i) providing a mixture consisiting of a blood sample obtained from the patient and a bacteria suspension, wherein the bacteria are antimicrobial peptide (AMP) sensitive,
(ii) incubating the mixture, and
(iii) determining the change in density of the mixture over time.

The bacteria comprised in the mixture provided in step (i) are preferably grown to log phase. In other words, the bacteria comprised in the mixture provided in step (i) preferably show an exponential growth. This exponential growth appears as a linear increase in optical density or extinction.

The mixture in step (i) may be provided by adding the blood sample obtained from the patient to the bacteria suspension or by adding the bacteria suspension to the blood sample obtained from the patient. Preferably, the mixture in step (i) is provided by adding the blood sample obtained from the patient to the bacteria suspension. The bacteria to which the blood sample is added or the bacteria added to the blood sample are preferably grown to log phase. In other words, the bacteria to which the blood sample is added or the bacteria added to the blood sample preferably show an exponential growth. This exponential growth appears as a linear increase in optical density or extinction. Preferably, the bacteria suspension added to the blood sample or to which the blood sample is added has an optical density at 600 nm of between 0.1 and 0.2, more preferably of between 0.12 and 0.18, and even more preferably of between 0.15 and 0.18. Preferably, the mixture provided in step (i) and/or at the beginning of the incubation phase/period in step (ii) has an optical density at 600 nm of between 0.1 and 0.3, more preferably of between 0.1 and 0.15, and even more preferably of between 0.12 and 0.15.

The mixture is further incubated in step (ii). Preferably, the mixture is incubated in step (ii) for between 0 and 4 hours, more preferably for between 0.5 and 2 hours, even more preferably for between 0.5 and 1 hour(s). For example, the mixture is incubated in step (ii) for 0 hours, for at least 0.5, 1, 2 or 3 hour(s), or for 4 hours. Particularly, the density of the mixture is determined immediately after the beginning of the incubation process/at the beginning of the incubation process (after 0 hours).

Preferably, the change in density is compared to a change in reference density over time.

In one embodiment, the change in reference density is the change in density determined by measuring a reference mixture of a blood sample obtained from a subject having an intact humoral immune system and a bacteria suspension.
The mixture as well as the reference mixture are preferably incubated in parallel/simultaneously and/or under the same experimental conditions (e.g. temperature, humidity, incubation time, starting cell density, dilution, cultivation/incubation type such as shaking cultivation/incubation). Further, the mixture as well as the reference mixture preferably have, when provided and/or at the beginning of the incubation process, the same bacteria density. Furthermore, the mixture as well as the reference mixture are preferably incubated for the same period of time.
It is preferred that the change in reference density of the reference mixture is determined by measuring at least at one first reference point in time and at least at one second reference point in time.
Preferably, the at least one first reference point in time is immediately after the beginning of the incubation process/at the beginning of the incubation process (time point 0 hours) or at least 0.5 hours, e.g. at least 0.5, 1, 2, 3, or 4 hour(s), after the beginning of the incubation process.
Preferably, the time period between the at least one first reference point in time and the at least one second reference point in time amounts to between 1 and 8 hours, preferably to between 2 and 6 hours, and more preferably to between 3 and 5 hours. For example, the time period between the at least one first reference point in time and the at least one second reference point in time amounts to at least 1, 2, 3, 4, 5, 6 or 7 hour(s), or 8 hour(s). The reference density may be determined at least at 2, 3, 4, 5, or 6 time points.
It is further preferred that said determining in step (iii) comprises determining the density of the mixture at least at one first point in time and at least at one second point in time.
Preferably, the at least one first point in time is immediately after the beginning of the incubation process/at the beginning of the incubation process (time point 0 hours), or at least 0.5 hours, e.g. at least 0.5, 1, 2, 3, or 4 hour(s), after the beginning of the incubation process. Preferably, the time period between the at least one first point in time and the at least one second point in time amounts to between 1 and 8 hours, preferably between 2 and 6 hours, more preferably between 3 and 5 hours. For example, the time period between the at least one first point in time and the at least one second point in time amounts to at least 1, 2, 3, 4, 5, 6 or 7 hour(s), or 8 hour(s). The density may be determined at least at 2, 3, 4, 5, or 6 time points. The mixture as well as the reference mixture are preferably tested at the same time points and/or time intervals.
It is also preferred that the difference between the at least one second point in time and the at least one first point in time is compared to the difference between the at least one second reference point in time and the at least one first reference point in time. In particular, the change in density is compared to a change in reference density over time by comparing the difference between the at least one second point in time and the at least one first point in time to the difference between the at least one second reference point in time and the at least one first reference point in time.
It is more preferred that
(i) a comparability of the difference between the at least one second point in time and the at least one first point in time to the difference between the at least one second reference point in time and the at least one first reference point in time indicates that the patient has an intact humoral immune system,
(ii) an increase of the difference between the at least one second point in time and the at least one first point in time to the difference between the at least one second reference point in time and the at least one first reference point in time indicates that the patient has an impaired humoral immune system, or
(iii) a decrease of the difference between the at least one second point in time and the at least one first point in time to the difference between the at least one second reference point in time and the at least one first reference point in time indicates that the patient has an intact humoral immune system.
Preferably, a comparability of the difference between the at least one second point in time and the at least one first point in time to the difference between the at least one second reference point in time and the at least one first reference point in time means a variation of between 0 and < 10%, e.g. 0, 0.1, 0.2, 0.3, 0.4, 0.5, 0.6, 0.7, 0.8, 0.9, 1, 2, 3, 4, 5, 6, 7, 8, 9, or 9.999%. Alternatively, a comparability preferably means a variation which is within the accuracy of a measurement. The accuracy of a measurement depends on the measurement method used. More preferably, the differences are identical.
Preferably, the increase of the difference between the at least one second point in time and the at least one first point in time to the difference between the at least one second reference point in time and the at least one first reference point in time is at least 0.5 times of the absolute amount of the difference between the at least one second reference point in time and the at least one first reference point in time.
More preferably, the increase of the difference between the at least one second point in time and the at least one first point in time to the difference between the at least one second reference point in time and the at least one first reference point in time is at least 1 times of the absolute amount of the difference between the at least one second reference point in time and the at least one first reference point in time.
Even more preferably, the increase of the difference between the at least one second point in time and the at least one first point in time to the difference between the at least one second reference point in time and the at least one first reference point in time is at least 2 times of the absolute amount of the difference between the at least one second reference point in time and the at least one first reference point in time.
Most preferably, the increase of the difference between the at least one second point in time and the at least one first point in time to the difference between the at least one second reference point in time and the at least one first reference point in time is at least 8 times of the absolute amount of the difference between the at least one second reference point in time and the at least one first reference point in time.
Preferably, the decrease of the difference between the at least one second point in time and the at least one first point in time to the difference between the at least one second reference point in time and the at least one first reference point in time is at least 0.2 times or at least 0.3 times of the absolute amount of the difference between the at least one second reference point in time and the at least one first reference point in time.
More preferably, the decrease of the difference between the at least one second point in time and the at least one first point in time to the difference between the at least one second reference point in time and the at least one first reference point in time is at least 0.4 times or at least 0.5 times of the absolute amount of the difference between the at least one second reference point in time and the at least one first reference point in time.
Even more preferably, the decrease of the difference between the at least one second point in time and the at least one first point in time to the difference between the at least one second reference point in time and the at least one first reference point in time is at least 0.8 times or at least 1 times of the absolute amount of the difference between the at least one second reference point in time and the at least one first reference point in time.

In one embodiment, the change in reference density is the change in density determined by measuring at least one reference sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 reference sample(s), of a mixture of a blood sample obtained from a subject having an intact humoral immune system and a bacteria suspension.
Said change in reference density over time may be an average change in reference density over time. It may be determined by measuring changes in reference densities over time and calculating the "average" value (e.g. mean, median or modal value) thereof. Preferably, the change in reference density over time has been measured/determined under the same experimental conditions (e.g. incubation time, starting cell density, dilution) than the change in density of the mixture over time measured/determined in step (iii). Preferably, the blood sample comprised in the reference sample is from the same source (e.g. serum or plasma sample) than the blood sample comprised in the mixture provided in step (i).
It is preferred that
an increase of the change in density over time compared to the change in reference density over time indicates that the patient has an impaired humoral immune system, or
a change in density over time which is comparable with or lower than the change in reference density over time indicates that the patient has an intact humoral immune system.
It is more preferred that a change in density over time exceeding the limit of the average change of reference density over time at least plus single standard deviation or at least plus double standard deviation indicates that the patient has an impaired humoral immune system.
It is even more preferred that a change in density over time exceeding the limit of the average change of reference density over time at least plus 2.5 times standard deviation or at least plus 4 times standard deviation indicates that the patient has an impaired humoral immune system.
It is most preferred that a change in density over time exceeding the limit of the average change of reference density over time at least plus 5 times standard deviation or at least plus 15 times standard deviation indicates that the patient has an impaired humoral immune system.
It is also more preferred that a change in density over time which does not exceed the limit of the average change of density over time plus single standard deviation indicates that the patient has an intact humoral immune system.
It is also even more preferred that a change in density over time which is comparable to the change of reference density over time indicates that the patient has an intact humoral immune system.
It is also most preferred that a change in density over time which is lower than the change in reference density over time (to any amount) indicates that the patient has an intact humoral immune system.
"Comparable" in this respect preferably means that the change in density over time and the change in reference density over time differ from each other between 0 and < 10%, e.g. 0, 0.1, 0.2, 0.3, 0.4, 0.5, 0.6, 0.7, 0.8, 0.9, 1, 2, 3, 4, 5, 6, 7, 8, 9, or 9.999%. Alternatively, "comparable" in this respect preferably means a variation which is within the accuracy of a measurement. The accuracy of a measurement depends on the measurement method used. More preferably, the changes in densities over time are identical.

In one embodiment, the change in reference density is the change in density determined by measuring a reference bacteria suspension (not mixed with a blood sample).
The mixture as well as the reference bacteria suspension are preferably incubated in parallel/simultaneously and/or under the same experimental conditions (e.g. temperature, humidity, incubation time, starting cell density, dilution, cultivation/incubation type such as shaking cultivation/incubation). Further, the mixture as well as the reference bacteria suspension preferably have, when provided and/or at the beginning of the incubation process, the same bacteria density. Furthermore, the mixture as well as the reference bacteria suspension are preferably incubated for the same period of time.
It is preferred that the change in reference density of the reference bacteria suspension is determined by measuring at least at one first reference point in time and at least at one second reference point in time.
Preferably, the at least one first reference point in time is immediately after the beginning of the incubation process/at the beginning of the incubation process (time point 0 hours), or at least 0.5 hours, e.g. at least 0.5, 1, 2, 3, or 4 hour(s), after the beginning of the incubation process.
Preferably, the time period between the at least one first reference point in time and the at least one second reference point in time amounts to between 1 and 8 hours, preferably to between 2 and 6 hours, and more preferably to between 3 and 5 hours. For example, the time period between the at least one first reference point in time and the at least one second reference point in time amounts to at least 1, 2, 3, 4, 5, 6 or 7 hour(s), or 8 hour(s). The reference density may be determined at least at 2, 3, 4, 5, or 6 time points.
It is further preferred that said determining in step (iii) comprises determining the density of the mixture at least at one first point in time and at least at one second point in time.
Preferably, the at least one first point in time is immediately after the beginning of the incubation process/at the beginning of the incubation process (time point 0 hours), or at least 0.5 hours, e.g. at least 0.5, 1, 2, 3, or 4 hour(s), after the beginning of the incubation process.
Preferably, the time period between the at least one first point in time and the at least one second point in time amounts to between 1 and 8 hours, preferably between 2 and 6 hours, more preferably between 3 and 5 hours. For example, the time period between the at least one first point in time and the at least one second point in time amounts to at least 1, 2, 3, 4, 5, 6 or 7 hour(s), or 8 hour(s). The density may be determined at least at 2, 3, 4, 5, or 6 time points.
The mixture as well as the reference bacteria suspension are preferably tested at the same time points and/or time intervals.
It is also preferred that the difference between the at least one second point in time and the at least one first point in time is compared to the difference between the at least one second reference point in time and the at least one first reference point in time. In particular, the change in density is compared to a change in reference density over time by comparing the difference between the at least one second point in time and the at least one first point in time to the difference between the at least one second reference point in time and the at least one first reference point in time.
It is more preferred that
(i) a comparability of the difference between the at least one second point in time and the at least one first point in time to the difference between the at least one second reference point in time and the at least one first reference point in time indicates that the patient has an impaired humoral immune system, or
(ii) a decrease of the difference between the at least one second point in time and the at least one first point in time to the difference between the at least one second reference point in time and the at least one first reference point in time indicates that the patient has an impaired humoral immune system or an intact humoral immune system.
It is even more preferred that
(i) a minor decrease of the difference between the at least one second point in time and the at least one first point in time to the difference between the at least one second reference point in time and the at least one first reference point in time indicates that the patient has an impaired humoral immune system, or
(ii) a substantial decrease of the difference between the at least one second point in time and the at least one first point in time to the difference between the at least one second reference point in time and the at least one first reference point in time indicates that the patient has an intact humoral immune system.
Preferably, a comparability of the difference between the at least one second point in time and the at least one first point in time to the difference between the at least one second reference point in time and the at least one first reference point in time means a variation of between 0 and < 10%, e.g. 0, 0.1, 0.2, 0.3, 0.4, 0.5, 0.6, 0.7, 0.8, 0.9, 1, 2, 3, 4, 5, 6, 7, 8, 9, or 9.999%. Alternatively, a comparability preferably means a variation which is within the accuracy of a measurement. The accuracy of a measurement depends on the measurement method used. More preferably, the differences are identical.
Preferably, the minor decrease of the difference between the at least one second point in time and the at least one first point in time to the difference between the at least one second reference point in time and the at least one first reference point in time is no more than 0.8 times or no more than 1 times of the absolute amount of the difference between the at least one second reference point in time and the at least one first reference point in time.
More preferably, the minor decrease of the difference between the at least one second point in time and the at least one first point in time to the difference between the at least one second reference point in time and the at least one first reference point in time is no more than 0.5 times or no more than 0.7 times of the absolute amount of the difference between the at least one second reference point in time and the at least one first reference point in time.
Even more preferably, the minor decrease of the difference between the at least one second point in time and the at least one first point in time to the difference between the at least one second reference point in time and the at least one first reference point in time is no more than 0.1 times or no more than 0.3 times of the absolute amount of the difference between the at least one second reference point in time and the at least one first reference point in time.
Preferably, the substantial decrease of the difference between the at least one second point in time and the at least one first point in time to the difference between the at least one second reference point in time and the at least one first reference point in time is at least > 1 times or at least 1.1 times of the absolute amount of the difference between the at least one second reference point in time and the at least one first reference point in time.
More preferably, the substantial decrease of the difference between the at least one second point in time and the at least one first point in time to the difference between the at least one second reference point in time and the at least one first reference point in time is at least 1.2 times of the absolute amount of the difference between the at least one second reference point in time and the at least one first reference point in time.
Even more preferably, the substantial decrease of the difference between the at least one second point in time and the at least one first point in time to the difference between the at least one second reference point in time and the at least one first reference point in time is at least 1.3 times of the absolute amount of the difference between the at least one second reference point in time and the at least one first reference point in time.

In one embodiment, the change in reference density is the change in density determined by measuring at least one reference sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 reference sample(s), of a bacteria suspension (not mixed with a blood sample).
Said change in reference density over time may be an average change in reference density over time. It may be determined by measuring changes in reference densities over time and calculating the "average" value (e.g. mean, median or modal value) thereof. Preferably, the change in reference density over time has been measured/determined under the same experimental conditions (e.g. incubation time, starting cell density, dilution) than the change in density of the mixture over time measured/determined in step (iii).
It is preferred that
a decrease of the change in density over time compared to the change in reference density over time indicates that the patient has an intact humoral immune system or an impaired humoral immune system, or
a change in density over time which is comparable with the change in reference density over time or higher than the change in reference density over time indicates that the patient has an impaired humoral immune system.
It is particularly preferred that
a substantial decrease of the change in density over time compared to the change in reference density over time indicates that the patient has an intact humoral immune system, or
a change in density over time which is lower to a small degree than the change in reference density over time indicates that the patient has an impaired humoral immune system.
It is more preferred that a substantial decrease of the change in density over time which is at least > 1 times the change in reference density over time indicates that the patient has an intact humoral immune system.
It is also more preferred that a substantial decrease of the change in density over time which is at least 1.1 times the change in reference density over time indicates that the patient has an intact humoral immune system.
It is even more preferred that a substantial decrease of the change in density over time which is at least 1.2 times the change in reference density over time indicates that the patient has an intact humoral immune system.
It is most preferred that a substantial decrease of the change in density over time which is at least 1.3 times the change in reference density over time indicates that the patient has an intact humoral immune system.
It is also more preferred that a lower decrease in the change in density over time which is no more than 0.8 times or no more than 1 times the change in reference density over time indicates that the patient has an impaired humoral immune system.
It is also even more preferred that a lower decrease in the change in density over time which is no more than 0.5 times or no more than 0.7 times the change in reference density over time indicates that the patient has an impaired humoral immune system.
It is also most preferred that a lower decrease in the change in density over time which is no more than 0.1 times or no more than 0.3 times the change in reference density over time indicates that the patient has an impaired humoral immune system.
"Comparable" in this respect preferably means that the change in density over time and the change in reference density over time differ from each other between 0 and < 10%, e.g. 0, 0.1, 0.2, 0.3, 0.4, 0.5, 0.6, 0.7, 0.8, 0.9, 1, 2, 3, 4, 5, 6, 7, 8, 9, or 9.999%. Alternatively, "comparable" in this respect preferably means a variation which is within the accuracy of a measurement. The accuracy of a measurement depends on the measurement method used. More preferably, the changes in densities over time are identical.

In one another embodiment, said determining comprises determining the density of the mixture at a first point in time and at least at one later point in time and comparing the densities determined at the different time points.
The change in density of the mixture over time is determined. The development of the change in density of the mixture over time allows to determine the status of the humoral immune system in the patient. In this case, no comparison to a change in reference density over time, e.g. from a parallel culture, is needed.
Preferably, the at least one first point in time is immediately after the beginning of the incubation process/at the beginning of the incubation process (time point 0 hours), or at least 0.5 hours, e.g. at least 0.5, 1, 2, 3, or 4 hour(s), after the beginning of the incubation process.
Preferably, the time period between the at least one first point in time and the at least one later point in time amounts to between 1 and 8 hours, preferably to between 2 and 6 hours, and more preferably to between 3 and 5 hours. For example, the time period between the at least one first point in time and the at least one later point in time amounts to at least 1, 2, 3, 4, 5, 6 or 7 hour(s), or 8 hour(s). The density may be determined/re(tested) at least at 2, 3, 4, 5, or 6 time points.
It is preferred that the density which
(i) increases over time indicates that the patient has a deteriorated, in particular an impaired, humoral immune system,
(ii) does not change over time indicates that the patient has an unchanged humoral immune system (e.g. a deteriorated, in particular an impaired, or an intact humoral immune system), or
(iii) decreases over time indicates that the patient has an improved humoral immune system.
Preferably, said increase is at least 20% or 30%, even more preferably at least 40% or 50%, and most preferably at least 80% or 100%, over time.
A density which "does not change over time" in this respect preferably means a variation of between 0 and < 10%, e.g. 0, 0.1, 0.2, 0.3, 0.4, 0.5, 0.6, 0.7, 0.8, 0.9, 1, 2, 3, 4, 5, 6, 7, 8, 9, or 9.999%. Alternatively, a density which "does not change over time" in this respect preferably means a variation which is within the accuracy of a measurement. The accuracy of a measurement depends on the measurement method used. More preferably, the densities have a variation of 0%.
Preferably, said decrease is at least 20% or 30%, even more preferably at least 40% or 50%, and most preferably at least 80% or 100%, over time.

It is preferred that the method of determining the state of the humoral immune system in a patient is carried out over a time period of between 4 and 10 hours, more preferably over a time period of between 4 and 8 hours, even more preferably over a time period of between 4 and 6 hours. For example, the method of determining the state of the humoral immune system in a patient is carried out over a time period of at least 4, 5, 6, 7, 8, 9 hours, or 10 hours.

It is also preferred that the patient whose status of the humoral immune system is determined is a patient suffering from a disease, in particular from a disease affecting the humoral immune system. Preferably, the patient whose status of the humoral immune system is determined is a patient suffering from a liver disease. More preferably, the liver disease is selected from the group consisting of liver cirrhosis, liver fibrosis, hepatic steatosis, and hepatitis virus infection, even more preferably hepatitis C virus infection, most preferably chronic hepatitis C virus infection.

It is further preferred that the patient whose status of the humoral immune system is determined is a patient that has undergone or undergoes a treatment of the disease, in particular a disease affecting the humoral immune system. Preferably, the disease is a liver disease. More preferably, the liver disease is selected from the group consisting of liver cirrhosis, liver fibrosis, hepatic steatosis, and hepatitis virus infection, even more preferably hepatitis C virus infection, most preferably chronic hepatitis C virus infection.

The treatment of the disease may be selected from the group consisting of drug administration, nutrition adaptation, chemotherapy, surgery, and radiotherapy. In a preferred embodiment, the patient suffers from a liver disease. The treatment of the liver disease may be selected from the group consisting of drug administration, surgery, nutritional intervention, neutralization of aetiological entities, abstinence from hepatotoxic substances (e.g. alcohol), and therapeutic phlebotomy.

The treatment of the disease such as liver disease preferably improves the state of the humoral immune system in the patient. For example, if the state of the humoral immune system in the patient was impaired before the therapeutic treatment, it may be intact after the therapeutic treatment or if the state of the humoral immune system in the patient was deteriorated before the therapeutic treatment, it may be intact after the therapeutic treatment. Under the treatment of the disease such as liver disease, the state of the humoral immune system in the patient may also worsen. In this case, the therapy form may be changed, e.g. the drug may be changed or the drug may be administered at a higher dose than administered before.

In a second aspect, the present invention relates to a method of determining the susceptibility of a patient to (develop) an infection comprising the steps of:
(i) providing a mixture consisiting of a blood sample obtained from the patient and a bacteria suspension, wherein the bacteria are antimicrobial peptide (AMP) sensitive,
(ii) incubating the mixture, and
(iii) determining the change in density of the mixture over time.

The bacteria comprised in the mixture provided in step (i) are preferably grown to log phase. In other words, the bacteria comprised in the mixture provided in step (i) preferably show an exponential growth. This exponential growth appears as a linear increase in optical density or extinction.

The mixture in step (i) may be provided by adding the blood sample obtained from the patient to the bacteria suspension or by adding the bacteria suspension to the blood sample obtained from the patient. Preferably, the mixture in step (i) is provided by adding the blood sample obtained from the patient to the bacteria suspension. The bacteria to which the blood sample is added or the bacteria added to the blood sample are preferably grown to log phase. In other words, the bacteria to which the blood sample is added or the bacteria added to the blood sample preferably show an exponential growth. This exponential growth appears as a linear increase in optical density or extinction. Preferably, the bacteria suspension added to the blood sample or to which the blood sample is added has an optical density at 600 nm of between 0.1 and 0.2, more preferably of between 0.12 and 0.18, and even more preferably of between 0.15 and 0.18. Preferably, the mixture provided in step (i) and/or at the beginning of the incubation phase/period in step (ii) has an optical density at 600 nm of between 0.1 and 0.3, more preferably of between 0.1 and 0.15, and even more preferably of between 0.12 and 0.15.

The mixture is further incubated in step (ii). Preferably, the mixture is incubated in step (ii) for between 0 and 4 hours, more preferably for between 0.5 and 2 hours, even more preferably for between 0.5 and 1 hour(s). For example, the mixture is incubated in step (ii) for 0 hours, for at least 0.5, 1, 2 or 3 hour(s), or for 4 hours. Particularly, the density of the mixture is determined immediately after the beginning of the incubation process/at the beginning of the incubation process (after 0 hours).

Preferably, the change in density is compared to a change in reference density over time.

In one embodiment, the change in reference density is the change in density determined by measuring a reference mixture consisiting of a blood sample obtained from a subject having no susceptibility to develop an infection (preferably (also) having an intact humoral immune system) and a bacteria suspension.
The mixture as well as the reference mixture are preferably incubated in parallel/simultaneously and/or under the same experimental conditions (e.g. temperature, humidity, incubation time, starting cell density, dilution, cultivation/incubation type such as shaking cultivation/incubation). Further, the mixture as well as the reference mixture preferably have, when provided and/or at the beginning of the incubation process, the same bacteria density. Furthermore, the mixture as well as the reference mixture are preferably incubated for the same period of time.
It is preferred that the change in reference density of the reference mixture is determined by measuring at least at one first reference point in time and at least at one second reference point in time.
Preferably, the at least one first reference point in time is immediately after the beginning of the incubation process/at the beginning of the incubation process (time point 0 hours), or at least 0.5 hours, e.g. at least 0.5, 1, 2, 3, or 4 hour(s), after the beginning of the incubation process.
Preferably, the time period between the at least one first reference point in time and the at least one second reference point in time amounts to between 1 and 8 hours, preferably to between 2 and 6 hours, and more preferably to between 3 and 5 hours. For example, the time period between the at least one first reference point in time and the at least one second reference point in time amounts to at least 1, 2, 3, 4, 5, 6 or 7 hour(s), or 8 hour(s). The reference density may be determined at least at 2, 3, 4, 5, or 6 time points.
It is further preferred that said determining in step (iii) comprises determining the density of the mixture at least at one first point in time and at least at one second point in time.
Preferably, the at least one first point in time is immediately after the beginning of the incubation process/at the beginning of the incubation process (time point 0 hours), or at least 0.5 hours, e.g. at least 0.5, 1, 2, 3, or 4 hour(s), after the beginning of the incubation process.
Preferably, the time period between the at least one first point in time and the at least one second point in time amounts to between 1 and 8 hours, preferably between 2 and 6 hours, more preferably between 3 and 5 hours. For example, the time period between the at least one first point in time and the at least one second point in time amounts to at least 1, 2, 3, 4, 5, 6 or 7 hour(s), or 8 hour(s). The density may be determined at least at 2, 3, 4, 5, or 6 time points.
The mixture as well as the reference mixture are preferably tested at the same time points and/or time intervals.
It is also preferred that the difference between the at least one second point in time and the at least one first point in time is compared to the difference between the at least one second reference point in time and the at least one first reference point in time. In particular, the change in density is compared to a change in reference density over time by comparing the difference between the at least one second point in time and the at least one first point in time to the difference between the at least one second reference point in time and the at least one first reference point in time.
It is more preferred that
(i) a comparability of the difference between the at least one second point in time and the at least one first point in time to the difference between the at least one second reference point in time and the at least one first reference point in time indicates that the patient has no susceptibility to develop an infection,
(ii) an increase of the difference between the at least one second point in time and the at least one first point in time to the difference between the at least one second reference point in time and the at least one first reference point in time indicates that the patient has a susceptibility to develop an infection, or
(iii) a decrease of the difference between the at least one second point in time and the at least one first point in time to the difference between the at least one second reference point in time and the at least one first reference point in time indicates that the patient has no susceptibility to develop an infection.
Preferably, a comparability of the difference between the at least one second point in time and the at least one first point in time to the difference between the at least one second reference point in time and the at least one first reference point in time means a variation of between 0 and < 10%, e.g. 0, 0.1, 0.2, 0.3, 0.4, 0.5, 0.6, 0.7, 0.8, 0.9, 1, 2, 3, 4, 5, 6, 7, 8, 9, or 9.999%. Alternatively, a comparability preferably means a variation which is within the accuracy of a measurement. The accuracy of a measurement depends on the measurement method used. More preferably, the differences are identical.
Preferably, the increase of the difference between the at least one second point in time and the at least one first point in time to the difference between the at least one second reference point in time and the at least one first reference point in time is at least 0.5 times of the absolute amount of the difference between the at least one second reference point in time and the at least one first reference point in time.
More preferably, the increase of the difference between the at least one second point in time and the at least one first point in time to the difference between the at least one second reference point in time and the at least one first reference point in time is at least 1 times of the absolute amount of the difference between the at least one second reference point in time and the at least one first reference point in time.
Even more preferably, the increase of the difference between the at least one second point in time and the at least one first point in time to the difference between the at least one second reference point in time and the at least one first reference point in time is at least 2 times of the absolute amount of the difference between the at least one second reference point in time and the at least one first reference point in time.
Most preferably, the increase of the difference between the at least one second point in time and the at least one first point in time to the difference between the at least one second reference point in time and the at least one first reference point in time is at least 8 times of the absolute amount of the difference between the at least one second reference point in time and the at least one first reference point in time.
Preferably, the decrease of the difference between the at least one second point in time and the at least one first point in time to the difference between the at least one second reference point in time and the at least one first reference point in time is at least 0.2 or 0.3 times of the absolute amount of the difference between the at least one second reference point in time and the at least one first reference point in time.
More preferably, the decrease of the difference between the at least one second point in time and the at least one first point in time to the difference between the at least one second reference point in time and the at least one first reference point in time is at least 0.4 or 0.5 times of the absolute amount of the difference between the at least one second reference point in time and the at least one first reference point in time.
Even more preferably, the decrease of the difference between the at least one second point in time and the at least one first point in time to the difference between the at least one second reference point in time and the at least one first reference point in time is at least 0.8 or 1 times of the absolute amount of the difference between the at least one second reference point in time and the at least one first reference point in time.

In one embodiment, the change in reference density is the change in density determined by measuring at least one reference sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 reference sample(s), of a mixture of a blood sample obtained from a subject having no susceptibility to develop an infection (preferably (also) having an intact humoral immune system) and a bacteria suspension.
Said change in reference density over time may be an average change in reference density over time. It may be determined by measuring changes in reference densities over time and calculating the "average" value (e.g. mean, median or modal value) thereof. Preferably, the change in reference density over time has been measured/determined under the same experimental conditions (e.g. incubation time, starting cell density, dilution) than the change in density of the mixture over time measured/determined in step (iii). Preferably, the blood sample comprised in the reference sample is from the same source (e.g. serum or plasma sample) than the blood sample comprised in the mixture provided in step (i).
It is preferred that
an increase of the change in density over time compared to the change in reference density over time indicates that the patient has a susceptibility to develop an infection, or
a change in density over time which is comparable with or lower than the change in reference density over time indicates that the patient has no susceptibility to develop an infection.
It is more preferred that a change in density over time exceeding the limit of the average change of reference density over time at least plus single standard deviation or at least plus double standard deviation indicates that the patient has an impaired humoral immune system.
It is even more preferred that a change in density over time exceeding the limit of the average change of reference density over time at least plus 2.5 times standard deviation or at least plus 4 times standard deviation indicates that the patient has an impaired humoral immune system.
It is most preferred that a change in density over time exceeding the limit of the average change of reference density over time at least plus 5 times standard deviation or at least plus 15 times standard deviation indicates that the patient has a susceptibility to develop an infection.
It is also more preferred that a change in density over time which does not exceed the limit of the average change of density over time plus single standard deviation indicates that the patient has an intact humoral immune system.
It is also even more preferred that a change in density over time which is comparable to the change of reference density over time indicates that the patient has an intact humoral immune system.
It is also most preferred that a change in density over time which is lower than the change in reference density over time (to any amount) indicates that the patient has no susceptibility to develop an infection.
"Comparable" in this respect preferably means that the change in density over time and the change in reference density over time differ from each other between 0 and < 10%, e.g. 0, 0.1, 0.2, 0.3, 0.4, 0.5, 0.6, 0.7, 0.8, 0.9, 1, 2, 3, 4, 5, 6, 7, 8, 9, or 9.999%. Alternatively, "comparable" in this respect preferably means a variation which is within the accuracy of a measurement. The accuracy of a measurement depends on the measurement method used. More preferably, the changes in densities over time are identical.

In one embodiment, the change in reference density is the change in density determined by measuring a reference bacteria suspension (not mixed with a blood sample).
The mixture as well as the reference bacteria suspension are preferably incubated in parallel/simultaneously and/or under the same experimental conditions (e.g. temperature, humidity, incubation time, starting cell density, dilution, cultivation/incubation type such as shaking cultivation/incubation). Further, the mixture as well as the reference bacteria suspension preferably have, when provided and/or at the beginning of the incubation process, the same bacteria density. Furthermore, the mixture as well as the reference bacteria suspension are preferably incubated for the same period of time.
It is preferred that the change in reference density of the reference bacteria suspension is determined by measuring at least at one first reference point in time and at least at one second reference point in time.
Preferably, the at least one first reference point in time is immediately after the beginning of the incubation process/at the beginning of the incubation process (time point 0 hours), or at least 0.5 hours, e.g. at least 0.5, 1, 2, 3, or 4 hour(s), after the beginning of the incubation process.
Preferably, the time period between the at least one first reference point in time and the at least one second reference point in time amounts to between 1 and 8 hours, preferably to between 2 and 6 hours, and more preferably to between 3 and 5 hours. For example, the time period between the at least one first reference point in time and the at least one second reference point in time amounts to at least 1, 2, 3, 4, 5, 6 or 7 hour(s), or 8 hour(s). The reference density may be determined at least at 2, 3, 4, 5, or 6 time points.
It is further preferred that said determining in step (iii) comprises determining the density of the mixture at least at one first point in time and at least at one second point in time.
Preferably, the at least one first point in time is immediately after the beginning of the incubation process/at the beginning of the incubation process (time point 0 hours), or at least 0.5 hours, e.g. at least 0.5, 1, 2, 3, or 4 hour(s), after the beginning of the incubation process.
Preferably, the time period between the at least one first point in time and the at least one second point in time amounts to between 1 and 8 hours, preferably between 2 and 6 hours, more preferably between 3 and 5 hours. For example, the time period between the at least one first point in time and the at least one second point in time amounts to at least 1, 2, 3, 4, 5, 6 or 7 hour(s), or 8 hour(s). The density may be determined at least at 2, 3, 4, 5, or 6 time points.
The mixture as well as the reference bacteria suspension are preferably tested at the same time points and/or time intervals.
It is also preferred that the difference between the at least one second point in time and the at least one first point in time is compared to the difference between the at least one second reference point in time and the at least one first reference point in time. In particular, the change in density over time is compared to a change in reference density over time by comparing the difference between the at least one second point in time and the at least one first point in time to the difference between the at least one second reference point in time and the at least one first reference point in time.
It is more preferred that
(i) a comparability of the difference between the at least one second point in time and the at least one first point in time to the difference between the at least one second reference point in time and the at least one first reference point in time indicates that the patient has a susceptibility to develop an infection, or
(ii) a decrease of the difference between the at least one second point in time and the at least one first point in time to the difference between the at least one second reference point in time and the at least one first reference point in time indicates that the patient has a susceptibility to develop an infection or no susceptibility to develop an infection.
It is even more preferred that
(i) a minor decrease of the difference between the at least one second point in time and the at least one first point in time to the difference between the at least one second reference point in time and the at least one first reference point in time indicates that the patient has a susceptibility to develop an infection, or
(ii) a substantial decrease of the difference between the at least one second point in time and the at least one first point in time to the difference between the at least one second reference point in time and the at least one first reference point in time indicates that the patient has no susceptibility to develop an infection.
Preferably, a comparability of the difference between the at least one second point in time and the at least one first point in time to the difference between the at least one second reference point in time and the at least one first reference point in time means a variation of between 0 and < 10%, e.g. 0, 0.1, 0.2, 0.3, 0.4, 0.5, 0.6, 0.7, 0.8, 0.9, 1, 2, 3, 4, 5, 6, 7, 8, 9, or 9.999%. Alternatively, a comparability preferably means a variation which is within the accuracy of a measurement. The accuracy of a measurement depends on the measurement method used. More preferably, the differences are identical.
Preferably, the minor decrease of the difference between the at least one second point in time and the at least one first point in time to the difference between the at least one second reference point in time and the at least one first reference point in time is no more than 0.8 times or no more than 1 times of the absolute amount of the difference between the at least one second reference point in time and the at least one first reference point in time.
More preferably, the minor decrease of the difference between the at least one second point in time and the at least one first point in time to the difference between the at least one second reference point in time and the at least one first reference point in time is no more than 0.5 times or no more than 0.7 times of the absolute amount of the difference between the at least one second reference point in time and the at least one first reference point in time.
Even more preferably, the minor decrease of the difference between the at least one second point in time and the at least one first point in time to the difference between the at least one second reference point in time and the at least one first reference point in time is no more than 0.1 times or no more than 0.3 times of the absolute amount of the difference between the at least one second reference point in time and the at least one first reference point in time.
Preferably, the substantial decrease of the difference between the at least one second point in time and the at least one first point in time to the difference between the at least one second reference point in time and the at least one first reference point in time is at least > 1 times or at least 1.1 times of the absolute amount of the difference between the at least one second reference point in time and the at least one first reference point in time.
More preferably, the substantial decrease of the difference between the at least one second point in time and the at least one first point in time to the difference between the at least one second reference point in time and the at least one first reference point in time is at least 1.2 times of the absolute amount of the difference between the at least one second reference point in time and the at least one first reference point in time.
Even more preferably, the substantial decrease of the difference between the at least one second point in time and the at least one first point in time to the difference between the at least one second reference point in time and the at least one first reference point in time is at least 1.3 times of the absolute amount of the difference between the at least one second reference point in time and the at least one first reference point in time.

In one embodiment, the change in reference density is the change in density determined by measuring at least one reference sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 reference sample(s), of a bacteria suspension (not mixed with a blood sample).
Said change in reference density over time may be an average change in reference density over time. It may be determined by measuring changes in reference densities over time and calculating the "average" value (e.g. mean, median or modal value) thereof. Preferably, the change in reference density over time has been measured/determined under the same experimental conditions (e.g. incubation time, starting cell density, dilution) than the change in density of the mixture over time measured/determined in step (iii).
It is preferred that
a decrease of the change in density over time compared to the change in reference density over time indicates that the patient has no susceptibility to develop an infection or a susceptibility to develop an infection, or
a change in density over time which is comparable with the change in reference density over time or higher than the change in reference density over time indicates that the patient has a susceptibility to develop an infection.
It is particularly preferred that
a substantial decrease of the change in density over time compared to the change in reference density over time indicates that the patient has no susceptibility to develop an infection, or
a change in density over time which is lower to a small degree than the change in reference density over time indicates that the patient has a susceptibility to develop an infection.
It is more preferred that a substantial decrease of the change in density over time which is at least >1 times the change in reference density over time indicates that the patient has no susceptibility to develop an infection.
It is also more preferred that a substantial decrease of the change in density over time which is at least 1.1 times the change in reference density over time indicates that the patient has no susceptibility to develop an infection.
It is even more preferred that a substantial decrease of the change in density over time which is at least 1.2 times the change in reference density over time indicates that the patient has no susceptibility to develop an infection.
It is most preferred that a substantial decrease of the change in density over time which is at least 1.3 times the change in reference density over time indicates that the patient has no susceptibility to develop an infection.
It is also more preferred that a lower decrease in the change in density over time which is no more than 0.8 times or no more than 1 times the change in reference density over time indicates that the patient has a susceptibility to develop an infection.
It is also even more preferred that a lower decrease in the change in density over time which is no more than 0.5 times or no more than 0.7 times the change in reference density over time indicates that the patient has a susceptibility to develop an infection.
It is also most preferred that a lower decrease in the change in density over time which is no more than 0.1 times or no more than 0.3 times the change in reference density over time has a susceptibility to develop an infection.
"Comparable" in this respect preferably means that the change in density over time and the change in reference density over time differ from each other between 0 and < 10%, e.g. 0, 0.1, 0.2, 0.3, 0.4, 0.5, 0.6, 0.7, 0.8, 0.9, 1, 2, 3, 4, 5, 6, 7, 8, 9, or 9.999%. Alternatively, "comparable" in this respect preferably means a variation which is within the accuracy of a measurement. The accuracy of a measurement depends on the measurement method used. More preferably, the changes in densities over time are identical.

In one another embodiment, said determining comprises determining the density of the mixture at a first point in time and at least at one later point in time and comparing the densities determined at the different time points.
The change in density of the mixture over time is determined. The development of the change in density of the mixture over time allows to determine the susceptibility of a patient to (develop) an infection. In this case, no comparison to a change in reference density over time, e.g. from a parallel culture, is needed.
Preferably, the at least one first point in time is immediately after the beginning of the incubation process/at the beginning of the incubation process (time point 0 hours), or at least 0.5 hours, e.g. at least 0.5, 1, 2, 3, or 4 hour(s), after the beginning of the incubation process.
Preferably, the time period between the at least one first point in time and the at least one later point in time amounts to between 1 and 8 hours, preferably to between 2 and 6 hours, and more preferably to between 3 and 5 hours. For example, the time period between the at least one first point in time and the at least one later point in time amounts to at least 1, 2, 3, 4, 5, 6 or 7 hour(s), or 8 hour(s). The density may be determined/re(tested) at least at 2, 3, 4, 5, or 6 time points.
It is preferred that the density which
(i) increases over time indicates that the patient has a higher susceptibility to develop an infection,
(ii) does not change over time indicates that the patient has an unchanged susceptibility to develop an infection (e.g. a susceptibility to develop an infection or no susceptibility to develop an infection), or
(iii) decreases over time indicates that the patient has a lower susceptibility to develop an infection.
Preferably, said increase is at least 20% or 30%, even more preferably at least 40% or 50%, and most preferably at least 80% or 100%, over time.
A density which "does not change over time" in this respect preferably means a variation of between 0 and < 10%, e.g. 0, 0.1, 0.2, 0.3, 0.4, 0.5, 0.6, 0.7, 0.8, 0.9, 1, 2, 3, 4, 5, 6, 7, 8, 9, or 9.999%. Alternatively, a density which "does not change over time" in this respect preferably means a variation which is within the accuracy of a measurement. The accuracy of a measurement depends on the measurement method used. More preferably, the densities have a variation of 0%.
Preferably, said decrease is at least 20% or 30%, even more preferably at least 40% or 50%, and most preferably at least 80% or 100%, over time.

It is preferred that the method of determining the susceptibility of a patient to (develop) an infection is carried out over a time period of between 4 and 10 hours, more preferably over a time period of between 4 and 8 hours, even more preferably over a time period of between 4 and 6 hours. For example, the method of determining the susceptibility of a patient to (develop) an infection is carried out over a time period of at least 4, 5, 6, 7, 8, 9 hours, or 10 hours.

Preferably, the infection is a severe infection. More preferably, the (severe) infection is selected from the group consisting of an urinary tract infection, a skin and soft tissue infection, an airway infection, a spontaneous bacterial peritonitis, a sepsis, and a gastrointestinal infection.

The patient who is determined as having a susceptibility to develop an infection is preferably a patient having a deteriorated, in particular an impaired, humoral immune system. The patient who is determined as having no susceptibility to develop an infection is preferably a patient having an intact or improved humoral immune system.

It is also preferred that the patient whose susceptibility to (develop) an infection is determined is a patient suffering from a disease. Preferably, the patient whose susceptibility to (develop) an infection is determined is a patient suffering from a liver disease. More preferably, the liver disease is selected from the group consisting of liver cirrhosis, liver fibrosis, hepatic steatosis, and hepatitis virus infection, even more preferably hepatitis C virus infection, most preferably chronic hepatitis C virus infection.

It is further preferred that the patient whose susceptibility to (develop) an infection is determined is a patient that has undergone or undergoes a treatment of the disease. Preferably, the disease is a liver disease. More preferably, the liver disease is selected from the group consisting of liver cirrhosis, liver fibrosis, hepatic steatosis, and hepatitis virus infection, even more preferably hepatitis C virus infection, most preferably chronic hepatitis C virus infection.

The treatment of the disease may be selected from the group consisting of drug administration, nutrition adaptation, chemotherapy, surgery, and radiotherapy. In a preferred embodiment, the patient suffers from a liver disease. The treatment of the liver disease may be selected from the group consisting of drug administration, surgery, nutritional intervention, neutralization of aetiological entities, abstinence from hepatotoxic substances (e.g. alcohol), and therapeutic phlebotomy.

The treatment of the disease such as liver disease preferably reduces the susceptibility of the patient to (develop) an infection. For example, if the patient had a susceptibility to (develop) an infection before the therapeutic treatment, it may have no susceptibility to develop an infection after the therapeutic treatment anymore. Under the treatment of the disease such as liver disease, the patient may also become susceptible to (develop) an infection. In this case, the therapy form may be changed, e.g. the drug may be changed or the drug may be administered at a higher dose than administered before.

In a third aspect, the present invention relates to a method of monitoring the state of the humoral immune system in a patient comprising the steps of:
(a) carrying out an incubation cycle comprising
   (i) providing a (first) mixture consisiting of a blood sample obtained from the patient and a bacteria suspension, wherein the bacteria are antimicrobial peptide (AMP) sensitive,
   (ii) incubating the (first) mixture, and
   (iii) determining the change in density of the (first) mixture over time, wherein said determining comprises determining the density of the (first) mixture at least at one first point in time and at least at one second point in time and comparing the densities determined at the different time points, and
(b) carrying out at least one further incubation cycle comprising
   (i) providing a (second) mixture consisiting of a blood sample obtained from the (same) patient and a bacteria suspension, wherein the bacteria are antimicrobial peptide (AMP) sensitive,
   (ii) incubating the (second) mixture, and
   (iii) determining the change in density of the (second) mixture over time, wherein said determining comprises determining the density of the (second) mixture at least at one third point in time and at least at one fourth point in time and comparing the densities determined at the different time points.

The bacteria comprised in the mixture provided in step (i) of (a) or (b) are preferably grown to log phase. In other words, the bacteria comprised in the mixture provided in step (i) of (a) or (b) preferably show an exponential growth. This exponential growth appears as a linear increase in optical density or extinction.

The mixture in step (i) of (a) or (b) may be provided by adding the blood sample obtained from the patient to the bacteria suspension or by adding the bacteria suspension to the blood sample obtained from the patient. Preferably, the mixture in step (i) of (a) or (b) is provided by adding the blood sample obtained from the patient to the bacteria suspension. The bacteria to which the blood sample is added or the bacteria added to the blood sample are preferably grown to log phase. In other words, the bacteria to which the blood sample is added or the bacteria added to the blood sample preferably show an exponential growth. This exponential growth appears as a linear increase in optical density or extinction. Preferably, the bacteria suspension added to the blood sample or to which the blood sample is added has an optical density at 600 nm of between 0.1 and 0.2, more preferably of between 0.12 and 0.18, and even more preferably of between 0.15 and 0.18. Preferably, the mixture provided in step (i) and/or at the beginning of the incubation phase/period in step (ii) has an optical density at 600 nm of between 0.1 and 0.3, more preferably of between 0.1 and 0.15, and even more preferably of between 0.12 and 0.15.

The mixture is further incubated in step (ii) of (a) or (b). Preferably, the mixture is incubated in step (ii) of (a) or (b) for between 0 and 4 hours, more preferably for between 0.5 and 2 hours, even more preferably for between 0.5 and 1 hour(s). For example, the mixture is incubated in step (ii) of (a) or (b) for 0 hours, for at least 0.5, 1, 2 or 3 hour(s), or for 4 hours. Particularly, the density of the mixture is determined immediately after the beginning of the incubation process/at the beginning of the incubation process (after 0 hours).

Preferably, the at least one first point in time is immediately after the beginning of the incubation process/at the beginning of the incubation process (time point 0 hours), or at least 0.5 hours, e.g. at least 0.5, 1, 2, 3, or 4 hour(s), after the beginning of the incubation process. Preferably, the time period between the at least one first point in time and the at least one second point in time amounts to between 1 and 8 hours, preferably to between 2 and 6 hours, and more preferably to between 3 and 5 hours. For example, the time period between the at least one first point in time and the at least one second point in time amounts to at least 1, 2, 3, 4, 5, 6 or 7 hour(s), or 8 hour(s). The density may be determined/re(tested) at least at 2, 3, 4, 5, or 6 time points.

Preferably, the at least one third point in time is immediately after the beginning of the incubation process/at the beginning of the incubation process (time point 0 hours), or at least 0.5 hours, e.g. at least 0.5, 1, 2, 3, or 4 hour(s), after the beginning of the incubation process. Preferably, the time period between the at least one third point in time and the at least one fourth point in time amounts to between 1 and 8 hours, preferably to between 2 and 6 hours, and more preferably to between 3 and 5 hours. For example, the time period between the at least one third point in time and the at least one fourth point in time amounts to at least 1, 2, 3, 4, 5, 6 or 7 hour(s), or 8 hour(s). The density may be determined/re(tested) at least at 2, 3, 4, 5, or 6 time points.

The time period between the incubation cycle in (a) and the at least one further incubation cycle in (b) preferably amounts to at least 1, 2, 3, 4, 5, 6, 7 day(s), 1, 2, 3, 4 week(s), 1, 2, 3, 4, 5, 6, 7, 8, 9, 10, 11, 12 month(s), 1, 2, 3, 4, 5, or 6 years.

The time period between the at least one second point in time and the at least one third point in time preferably amounts to at least 1, 2, 3, 4, 5, 6, 7 day(s), 1, 2, 3, 4 week(s), 1, 2, 3, 4, 5, 6, 7, 8, 9, 10, 11, 12 month(s), 1, 2, 3, 4, 5, or 6 years.

The above-mentioned method particularly allows the monitoring of the state of the humoral immune system in a patient over time of care. The time of care in this respect specifically means the time the patient is under medical observation. This medical observation time may be necessary/required in view of the disease the patient suffers from and/or in order to evaluate the cause of treatment or treatment success of a patient suffering from a disease.

The (first) mixture in (a) as well as the (second) mixture in (b) are preferably incubated under the same experimental conditions (e.g. temperature, humidity, incubation time, starting cell density, dilution, cultivation/incubation type such as shaking cultivation/incubation). Further, the (first) mixture in (a) as well as the (second) mixture in (b) preferably have, when provided and/or at the beginning of the incubation process, the same bacteria density. Furthermore, the (first) mixture in (a) as well as the (second) mixture in (b) are preferably incubated for the same period of time.

It is preferred that the difference between the at least one fourth point in time and the at least one third point in time is compared to the difference between the at least one second point in time and the at least one first point in time.

It is more preferred that,
(i) a comparability of the difference between the at least one fourth point in time and the at least one third point in time to the difference between the at least one second point in time and the at least one first point in time indicates that the patient has an unchanged humoral immune system (e.g. a deteriorated, in particular an impaired, or an intact humoral immune system),
(ii) an increase of the difference between the at least one fourth point in time and the at least one third point in time to the difference between the at least one second point in time and the at least one first point in time indicates that the patient has a deteriorated, in particular an impaired, humoral immune system, or
(iii) a decrease of the difference between the at least one fourth point in time and the at least one third point in time to the difference between the at least one second point in time and the at least one first point in time indicates that the patient has an improved humoral immune system.
Preferably, a comparability of the difference between the at least one fourth point in time and the at least one third point in time to the difference between the at least one second point in time and the at least one first point in time means a variation of between 0 and < 10%, e.g. 0, 0.1, 0.2, 0.3, 0.4, 0.5, 0.6, 0.7, 0.8, 0.9, 1, 2, 3, 4, 5, 6, 7, 8, 9, or 9.999%. Alternatively, a comparability preferably means a variation which is within the accuracy of a measurement. The accuracy of a measurement depends on the measurement method used. More preferably, the differences are identical.
Preferably, said increase is at least 20% or 30%, even more preferably at least 40% or 50%, and most preferably at least 80% or 100%, over time.
Preferably, said decrease is at least 20% or 30%, even more preferably at least 40% or 50%, and most preferably at least 80% or 100%, over time.

It is preferred that the method of monitoring the state of the humoral immune system in a patient is carried out over a time period (time of care) of between 1 day and 6 years, more preferably over a time period (time of care) of between 1 day and 2 years, even more preferably over a time period (time of care) of between 1 week and 1 year, and most preferably over a time period of between 1 month and 1 year. For example, the method of monitoring the state of the humoral immune system in a patient is carried out over a time period (time of care) of at least 1, 2, 3, 4, 5, 6, 7 day(s), 1, 2, 3, 4 week(s), 1, 2, 3, 4, 5, 6, 7, 8, 9, 10, 11, 12 month(s), 1, 2, 3, 4, 5, or 6 years.

It is also preferred that the patient whose status of the humoral immune system is monitored (over time of care) is a patient suffering from a disease, in particular from a disease affecting the humoral immune system. Preferably, the patient whose status of the humoral immune system is monitored (over time of care) is a patient suffering from a liver disease. More preferably, the liver disease is selected from the group consisting of liver cirrhosis, liver fibrosis, hepatic steatosis, and hepatitis virus infection, even more preferably hepatitis C virus infection, most preferably chronic hepatitis C virus infection.

It is further preferred that the patient whose status of the humoral immune system is monitored (over time of care) is a patient that has undergone or undergoes a treatment of the disease, in particular a disease affecting the humoral immune system. Preferably, the disease is a liver disease. More preferably, the liver disease is selected from the group consisting of liver cirrhosis, liver fibrosis, hepatic steatosis, and hepatitis virus infection, even more preferably hepatitis C virus infection, most preferably chronic hepatitis C virus infection.

The treatment of the disease may be selected from the group consisting of drug administration, nutrition adaptation, chemotherapy, surgery, and radiotherapy. In a preferred embodiment, the patient suffers from a liver disease. The treatment of the liver disease may be selected from the group consisting of drug administration, surgery, nutritional intervention, neutralization of aetiological entities, abstinence from hepatotoxic substances (e.g. alcohol), and therapeutic phlebotomy.

The treatment of the disease such as liver disease preferably improves the state of the humoral immune system of the patient (during time of care). For example, if the state of the humoral immune system of the patient was impaired before the therapeutic treatment, it may be intact after the therapeutic treatment or if the state of the humoral immune system of the patient was deteriorated before the therapeutic treatment, it may be intact after the therapeutic treatment. Under the treatment of the disease such as liver disease, the state of the humoral immune system of the patient may also worsen. In this case, the therapy form may be changed, e.g. the drug may be changed or the drug may be administered at a higher dose than administered before.

It is particularly preferred that the patient has undergone or undergoes a therapeutic treatment of the disease, e.g. liver disease,
within/during the period after the at least one second point in time and before the at least one third point in time,
in the period before the at least one first point in time,
in the period before the at least one third point in time, and/or
in the period after the at least one second point in time.
In case that the patient has undergone or undergoes a therapeutic treatment of the disease, e.g. liver disease, in the period after the at least one second point in time, the treatment success between the incubation cycle in (a) and (b) can be determined (measurements at the at least one first point in time and at the at least one second point in time without treatment/medication, measurements at the at least one third point in time and at the at least one fourth point in time with treatment/medication).
For example, the patient may be a patient suffering from a (chronic) hepatitis C infection and the treatment after at least the second point in time is an antiviral treatment. Due to the antiviral treatment, the status of the humoral immune system improves in the patient.

In a fourth aspect, the present invention relates to a method of monitoring the susceptibility of a patient to (develop) an infection comprising the steps of:
(a) carrying out an incubation cycle comprising
   (i) providing a (first) mixture consisting of a blood sample obtained from the patient and a bacteria suspension, wherein the bacteria are antimicrobial peptide (AMP) sensitive,
   (ii) incubating the (first) mixture, and
   (iii) determining the change in density of the (first) mixture over time, wherein said determining comprises determining the density of the (first) mixture at least at one first point in time and at least at one second point in time and comparing the densities determined at the different time points, and
(b) carrying out at least one further incubation cycle comprising
   (i) providing a (second) mixture consisting of a blood sample obtained from the (same) patient and a bacteria suspension, wherein the bacteria are antimicrobial peptide (AMP) sensitive,
   (ii) incubating the (second) mixture, and
   (iii) determining the change in density of the (second) mixture over time, wherein said determining comprises determining the density of the (second) mixture at least at one third point in time and at least at one fourth point in time and comparing the densities determined at the different time points.

The bacteria comprised in the mixture provided in step (i) of (a) or (b) are preferably grown to log phase. In other words, the bacteria comprised in the mixture provided in step (i) of (a) or (b) preferably show an exponential growth. This exponential growth appears as a linear increase in optical density or extinction.

The mixture in step (i) of (a) or (b) may be provided by adding the blood sample obtained from the patient to the bacteria suspension or by adding the bacteria suspension to the blood sample obtained from the patient. Preferably, the mixture in step (i) of (a) or (b) is provided by adding the blood sample obtained from the patient to the bacteria suspension. The bacteria to which the blood sample is added or the bacteria added to the blood sample are preferably grown to log phase. In other words, the bacteria to which the blood sample is added or the bacteria added to the blood sample preferably show an exponential growth. This exponential growth appears as a linear increase in optical density or extinction. Preferably, the bacteria suspension added to the blood sample or to which the blood sample is added has an optical density at 600 nm of between 0.1 and 0.2, more preferably of between 0.12 and 0.18, and even more preferably of between 0.15 and 0.18. Preferably, the mixture provided in step (i) and/or at the beginning of the incubation phase/period in step (ii) has an optical density at 600 nm of between 0.1 and 0.3, more preferably of between 0.1 and 0.15, and even more preferably of between 0.12 and 0.15.

The mixture is further incubated in step (ii) of (a) or (b). Preferably, the mixture is incubated in step (ii) of (a) or (b) for between 0 and 4 hours, more preferably for between 0.5 and 2 hours, even more preferably for between 0.5 and 1 hour(s). For example, the mixture is incubated in step (ii) of (a) or (b) for 0 hours, for at least 0.5, 1, 2 or 3 hour(s), or for 4 hours. Particularly, the density of the mixture is determined immediately after the beginning of the incubation process/at the beginning of the incubation process (after 0 hours).

Preferably, the at least one first point in time is immediately after the beginning of the incubation process/at the beginning of the incubation process (time point 0 hours), or at least 0.5 hours, e.g. at least 0.5, 1, 2, 3, or 4 hour(s), after the beginning of the incubation process. Preferably, the time period between the at least one first point in time and the at least one second point in time amounts to between 1 and 8 hours, preferably to between 2 and 6 hours, and more preferably to between 3 and 5 hours. For example, the time period between the at least one first point in time and the at least one second point in time amounts to at least 1, 2, 3, 4, 5, 6 or 7 hour(s), or 8 hour(s). The density may be determined/re(tested) at least at 2, 3, 4, 5, or 6 time points.

Preferably, the at least one third point in time is immediately after the beginning of the incubation process/at the beginning of the incubation process (time point 0 hours), or at least 0.5 hours, e.g. at least 0.5, 1, 2, 3, or 4 hour(s), after the beginning of the incubation process. Preferably, the time period between the at least one third point in time and the at least one fourth point in time amounts to between 1 and 8 hours, preferably to between 2 and 6 hours, and more preferably to between 3 and 5 hours. For example, the time period between the at least one third point in time and the at least one fourth point in time amounts to at least 1, 2, 3, 4, 5, 6 or 7 hour(s), or 8 hour(s). The density may be determined/re(tested) at least at 2, 3, 4, 5, or 6 time points.

The time period between the incubation cycle in (a) and the at least one further incubation cycle in (b) preferably amounts to at least 1, 2, 3, 4, 5, 6, 7 day(s), 1, 2, 3, 4 week(s), 1, 2, 3, 4, 5, 6, 7, 8, 9, 10, 11, 12 month(s), 1, 2, 3, 4, 5, or 6 years.

The time period between the at least one second point in time and the at least one third point in time preferably amounts to at least 1, 2, 3, 4, 5, 6, 7 day(s), 1, 2, 3, 4 week(s), 1, 2, 3, 4, 5, 6, 7, 8, 9, 10, 11, 12 month(s), 1, 2, 3, 4, 5, or 6 years.

The above-mentioned method particularly allows the monitoring of the susceptibility of the patient to (develop) an infection over time of care. The time of care in this respect specifically means the time the patient is under medical observation. This medical observation time may be necessary/required in view of the disease the patient suffers from and/or in order to evaluate the cause of treatment or treatment success of a patient suffering from a disease.

The (first) mixture in (a) as well as the (second) mixture in (b) are preferably incubated under the same experimental conditions (e.g. temperature, humidity, incubation time, starting cell density, dilution, cultivation/incubation type such as shaking cultivation/incubation). Further, the (first) mixture in (a) as well as the (second) mixture in (b) preferably have, when provided and/or at the beginning of the incubation process, the same bacteria density. Furthermore, the (first) mixture in (a) as well as the (second) mixture in (b) are preferably incubated for the same period of time.

It is preferred that the difference between the at least one fourth point in time and the at least one third point in time is compared to the difference between the at least one second point in time and the at least one first point in time.

It is more preferred that,
(i) a comparability of the difference between the at least one fourth point in time and the at least one third point in time to the difference between the at least one second point in time and the at least one first point in time indicates that the patient has an unchanged susceptibility to develop an infection (e.g. a susceptibility to develop an infection or no susceptibility to develop an infection),
(ii) an increase of the difference between the at least one fourth point in time and the at least one third point in time to the difference between the at least one second point in time and the at least one first point in time indicates that the patient has a higher susceptibility to develop an infection, or
(iii) a decrease of the difference between the at least one fourth point in time and the at least one third point in time to the difference between the at least one second point in time and the at least one first point in time indicates that the patient has a lower susceptibility to develop an infection.
Preferably, a comparability of the difference between the at least one fourth point in time and the at least one third point in time to the difference between the at least one second point in time and the at least one first point in time means a variation of between 0 and < 10%, e.g. 0, 0.1, 0.2, 0.3, 0.4, 0.5, 0.6, 0.7, 0.8, 0.9, 1, 2, 3, 4, 5, 6, 7, 8, 9, or 9.999%. Alternatively, a comparability preferably means a variation which is within the accuracy of a measurement. The accuracy of a measurement depends on the measurement method used. More preferably, the differences are identical.
Preferably, said increase is at least 20% or 30%, even more preferably at least 40% or 50%, and most preferably at least 80% or 100%, over time.
Preferably, said decrease is at least 20% or 30%, even more preferably at least 40% or 50%, and most preferably at least 80% or 100%, over time.

It is preferred that the method of monitoring the susceptibility of a patient to (develop) an infection is carried out over a time period (time of care) of between 1 day and 6 years, more preferably over a time period (time of care) of between 1 day and 2 years, even more preferably over a time period (time of care) of between 1 week and 1 year, and most preferably over a time period of between 1 month and 1 year. For example, the method of monitoring the susceptibility of a patient to (develop) an infection is carried out over a time period (time of care) of at least 1, 2, 3, 4, 5, 6, 7 day(s), 1, 2, 3, 4 week(s), 1, 2, 3, 4, 5, 6, 7, 8, 9, 10, 11, 12 month(s), 1, 2, 3, 4, 5, or 6 years.

Preferably, the infection is a severe infection. More preferably, the (severe) infection is selected from the group consisting of an urinary tract infection, a skin and soft tissue infection, an airway infection, a spontaneous bacterial peritonitis, a sepsis, and a gastrointestinal infection.

The patient whose susceptibility to (develop) an infection is monitored may be a patient having an intact, an improved, or a deteriorated, in particular an impaired, humoral immune system. The patient who is determined as having a susceptibility to (develop) an infection during monitoring is preferably a patient having a deteriorated, in particular an impaired, humoral immune system. The patient who is determined as having no susceptibility to develop an infection during monitoring is preferably a patient having an intact or improved humoral immune system.

It is also preferred that the patient whose susceptibility to (develop) an infection is monitored (over time of care) is a patient suffering from a disease, in particular from a disease affecting the humoral immune system. Preferably, the patient whose susceptibility to (develop) an infection is monitored (over time of care) is a patient suffering from a liver disease. More preferably, the liver disease is selected from the group consisting of liver cirrhosis, liver fibrosis, hepatic steatosis, and hepatitis virus infection, even more preferably hepatitis C virus infection, most preferably chronic hepatitis C virus infection.

It is further preferred that the patient whose susceptibility to (develop) an infection is monitored (over time of care) is a patient that has undergone or undergoes a treatment of the disease, in particular a disease affecting the humoral immune system. Preferably, the disease is a liver disease. More preferably, the liver disease is selected from the group consisting of liver cirrhosis, liver fibrosis, hepatic steatosis, and hepatitis virus infection, even more preferably hepatitis C virus infection, most preferably chronic hepatitis C virus infection.

The treatment of the disease may be selected from the group consisting of drug administration, nutrition adaptation, chemotherapy, surgery, and radiotherapy. In a preferred embodiment, the patient suffers from a liver disease. The treatment of the liver disease may be selected from the group consisting of drug administration, surgery, nutritional intervention, neutralization of aetiological entities, abstinence from hepatotoxic substances (e.g. alcohol), and therapeutic phlebotomy.

The treatment of the disease such as liver disease preferably reduces the susceptibility of the patient to (develop) an infection. For example, if the patient had a susceptibility to (develop) an infection before the therapeutic treatment, it may have no susceptibility to develop an infection after the therapeutic treatment anymore. Under the treatment of the disease such as liver disease, the patient may also become susceptible to (develop) an infection. In this case, the therapy form may be changed, e.g. the drug may be changed or the drug may be administered at a higher dose than administered before.

It is particularly preferred that the patient has undergone or undergoes a therapeutic treatment of the disease, e.g. liver disease,
within/during the period after the at least one second point in time and before the at least one third point in time,
in the period before the at least one first point in time,
in the period before the at least one third point in time, and/or
in the period after the at least one second point in time.

It is preferred that the blood sample used in the method of the first to fourth aspect of the present invention is a whole blood or a blood fraction sample. It is more preferred that the blood fraction sample is a serum, plasma, or blood cell sample. The blood cell sample may comprise or consist of erythrocytes, leukocytes, and/or thrombocytes.
With respect to plasma, it is particularly preferred that the plasma is not EDTA-plasma. The plasma may be, however, lithium heparin-plasma or sodium citrate-plasma.

It is further preferred that, the density determined/measured in the method of the first to fourth aspect of the present invention is the optical density or the number of bacteria per volume unit. While the number of bacteria per volume unit may be determined by counting (e.g. using a counting chamber), the optical density may be determined by light scattering techniques, e.g. via the McFahrland Turbidity Standards or using a spectrophotometer. The optical density is preferably measured at a wave length of between 400 and 800 nm, more preferably at a wave length of 600 nm.

It is also preferred that the antimicrobial peptide (AMP) sensitive bacteria are gram-negative and/or aerobic bacteria. It is more preferred that the antimicrobial peptide (AMP) sensitive bacteria are *E. coli* bacteria. It is even more preferred that the antimicrobial peptide (AMP) sensitive bacteria are *E. coli* XL1 blue.

In a fifth aspect, the present invention relates to the use of a bacteria suspension to determine the state of the humoral immune system in a patient, wherein the bacteria suspension and the blood sample form a mixture consisting of the bacteria suspension and the blood sample, and wherein the bacteria are antimicrobial peptide (AMP) sensitive.

The patient may suffer from a disease, in particular from a disease affecting the humoral immune system. Preferably, the disease is a liver disease. More preferably, the liver disease is selected from the group consisting of liver cirrhosis, liver fibrosis, hepatic steatosis, and hepatitis virus infection, even more preferably hepatitis C virus infection, most preferably chronic hepatitis C virus infection.

Preferably, the antimicrobial peptide (AMP) sensitive bacteria are gram-negative and/or aerobic bacteria. More preferably, the antimicrobial peptide (AMP) sensitive bacteria are E. *coli* bacteria. Even more preferably, the antimicrobial peptide (AMP) sensitive bacteria are E. *coli* XL1 blue.

As to further preferred embodiments, it is referred to the first aspect of the present invention.

In a sixth aspect, the present invention relates to the use of a bacteria suspension to determine the susceptibility of a patient to (develop) an infection, wherein the bacteria suspension and the blood sample form a mixture consisting of the bacteria suspension and the blood sample, and wherein the bacteria are antimicrobial peptide (AMP) sensitive.

The patient may be a patient having an intact humoral immune system or a patient having a deteriorated, in particular an impaired, humoral immune system. The patient may suffer from a disease. Preferably, the disease is a liver disease. More preferably, the liver disease is selected from the group consisting of liver cirrhosis, liver fibrosis, hepatic steatosis, and hepatitis virus infection, even more preferably hepatitis C virus infection, most preferably chronic hepatitis C virus infection.

Preferably, the antimicrobial peptide (AMP) sensitive bacteria are gram-negative and/or aerobic bacteria. More preferably, the antimicrobial peptide (AMP) sensitive bacteria are E. *coli* bacteria. Even more preferably, the antimicrobial peptide (AMP) sensitive bacteria are E. *coli* XL1 blue.

Preferably, the infection is a severe infection. More preferably, the (severe) infection is selected from the group consisting of an urinary tract infection, a skin and soft tissue infection, an airway infection, a spontaneous bacterial peritonitis, a sepsis, and a gastrointestinal infection.

As to further preferred embodiments, it is referred to the second aspect of the present invention.

In a seventh aspect, the present invention relates to the use of a bacteria suspension to monitor the state of the humoral immune system in a patient, wherein the bacteria suspension and the blood sample form a mixture consisting of the bacteria suspension and the blood sample, and wherein the bacteria are antimicrobial peptide (AMP) sensitive.

The patient may suffer from a disease, in particular from a disease affecting the humoral immune system. Preferably, the disease is a liver disease. More preferably, the liver disease is selected from the group consisting of liver cirrhosis, liver fibrosis, hepatic steatosis, and hepatitis virus infection, even more preferably hepatitis C virus infection, most preferably chronic hepatitis C virus infection.

Preferably, the antimicrobial peptide (AMP) sensitive bacteria are gram-negative and/or aerobic bacteria. More preferably, the antimicrobial peptide (AMP) sensitive bacteria are E. *coli* bacteria. Even more preferably, the antimicrobial peptide (AMP) sensitive bacteria are E. *coli* XL1 blue.

As to further preferred embodiments, it is referred to the third aspect of the present invention.

In an eight aspect, the present invention relates to the use of a bacteria suspension to monitor the susceptibility of a patient to (develop) an infection, wherein the bacteria suspension and the blood sample form a mixture consisting of the bacteria suspension and the blood sample, and wherein the bacteria are antimicrobial peptide (AMP) sensitive.

The patient may be a patient having an intact humoral immune system or a patient having a deteriorated, in particular an impaired, humoral immune system. The patient may suffer from a disease. Preferably, the disease is a liver disease. More preferably, the liver disease is selected from the group consisting of liver cirrhosis, liver fibrosis, hepatic steatosis, and hepatitis virus infection, even more preferably hepatitis C virus infection, most preferably chronic hepatitis C virus infection.

Preferably, the antimicrobial peptide (AMP) sensitive bacteria are gram-negative and/or aerobic bacteria. More preferably, the antimicrobial peptide (AMP) sensitive bacteria are E. *coli* bacteria. Even more preferably, the antimicrobial peptide (AMP) sensitive bacteria are E. *coli* XL1 blue.

Preferably, the infection is a severe infection. More preferably, the (severe) infection is selected from the group consisting of an urinary tract infection, a skin and soft tissue infection, an airway infection, a spontaneous bacterial peritonitis, a sepsis, and a gastrointestinal infection.

As to further preferred embodiments, it is referred to the fourth aspect of the present invention.

In a ninth aspect, the present invention relates to the use of a kit for determining the state of the humoral immune system in a patient, the susceptibility of a patient to develop an infection, monitoring the state of the humoral immune system in a patient, and/or monitoring the susceptibility of a patient to develop an infection consisting of: (i) antimicrobial peptide (AMP) sensitive bacteria, (ii) means for sampling blood from a patient, and/or (iii) means for preparing a suspension of said bacteria and/or cultivating the suspension of said bacteria and/or a mixture consisting of the suspension of said bacteria and the blood sample.

The kit is useful for determining the state of the humoral immune system in a patient, the susceptibility of a patient to (develop) an infection, monitoring the state of the humoral immune system in a patient, and/or monitoring the susceptibility of a patient to (develop) an infection. In particular, the kit is useful for carrying out the method according to the first to fourth aspect of the present invention.

The patient may suffer from a disease, in particular a disease affecting the humoral immune system. Preferably, the disease is a liver disease. More preferably, the liver disease is selected from the group consisting of liver cirrhosis, liver fibrosis, hepatic steatosis, and hepatitis virus infection, even more preferably hepatitis C virus infection, most preferably chronic hepatitis C virus infection.

Preferably, the antimicrobial peptide (AMP) sensitive bacteria are gram-negative and/or aerobic bacteria. More preferably, the antimicrobial peptide (AMP) sensitive bacteria are E. *coli* bacteria. Even more preferably, the antimicrobial peptide (AMP) sensitive bacteria are E. *coli* XL1 blue.

Preferably, the infection is a severe infection. More preferably, the (severe) infection is selected from the group consisting of an urinary tract infection, a skin and soft tissue infection, an airway infection, a spontaneous bacterial peritonitis, a sepsis, and a gastrointestinal infection.

The kit may further comprise
(i) a container, and/or
(ii) a data carrier.
Said data carrier may be a non-electronical data carrier, e.g. a graphical data carrier such as an information leaflet, an information sheet, a bar code or an access code, or an electronical data carrier such as a floppy disk, a compact disk (CD), a digital versatile disk (DVD), a microchip or another semiconductor-based electronical data carrier. The access code may allow the access to a database, e.g. an internet database, a centralized, or a decentralized database. The access code may also allow access to an application software that causes a computer to perform tasks for computer users or a mobile app which is a software designed to run on smartphones and other mobile devices.
Said data carrier may further comprise one or more reference densities referred to herein.
In case that the data carrier comprises an access code which allows the access to a database, said one or more reference densities are deposited in this database.
In addition, the data carrier may comprise information or instructions on how to carry out the method according to the first to fourth aspect of the present invention.

Said kit may also comprise materials desirable from a commercial and user standpoint including (a) culture medium(media), (a) buffer(s), (a) reagent(s) and/or (a) diluent(s) for determining the reference density mentioned above.

Various modifications and variations of the invention will be apparent to those skilled in the art without departing from the scope of invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art in the relevant fields are intended to be covered by the present invention.

### BRIEF DESCRIPTION OF THE FIGURES

The following figures and examples are merely illustrative of the present invention and should not be construed to limit the scope of the invention as indicated by the appended claims in any way.

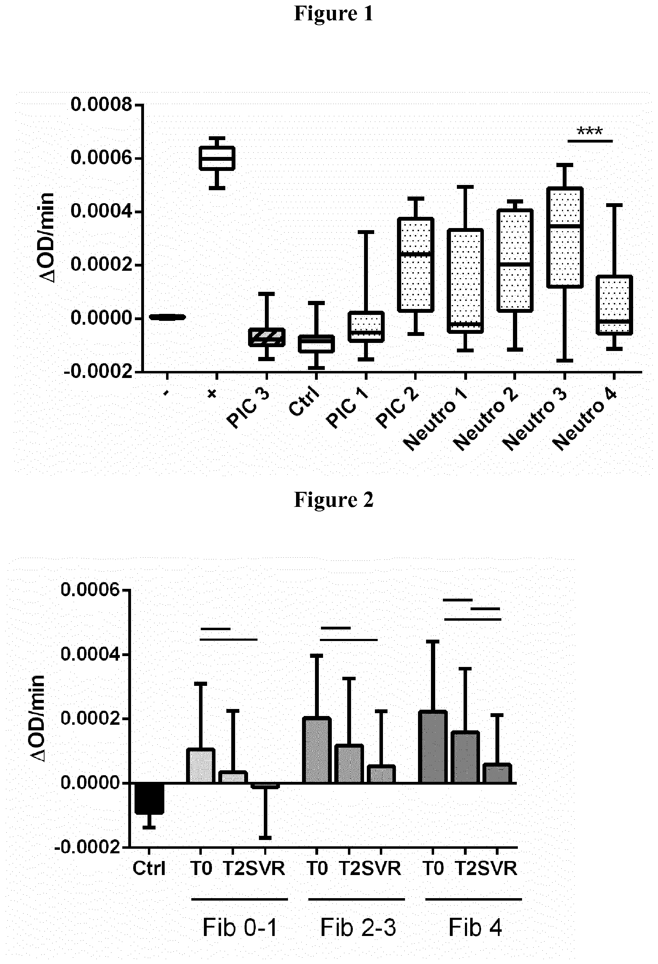

**FIGURE 1****:** Comparison of different liver disease cohorts compared to healthy controls. Cohort PIC 1 are cirrhotic patients with alcohol as aetiological entity; Cohort PIC 2 are cirrhotic patients with chronic hepatitis C infection as aetiological entitiy; Cohort PIC 3 are cirrhotic patients with a variety of causing factors including NALFD, hemochromatosis, primary biliary cirrhosis, chronic hepatitis B infection, primary sclerosing cholangitis, Morbus Wilson and unclear reasons; Cohort NEUTRO 1 are patients with chronic hepatitis C infection and no or mild liver fibrosis; Cohort NEUTRO 2 are patients with chronic hepatitis C infection and advanced liver fibrosis; Cohort NEUTRO 3 are patient with chronic hepatitis C infection and cirrhosis; Cohort NEUTRO 4 are patients from NEUTRO 3 after successful treatment of the hepatitis C infection. Groups with significantly impaired Growth retardation capacity are depicted with dotted boxes, cohorts with normal capacity with dashed gray boxes and healthy controls with blank boxes. +/- are technical controls to monitor growth conditions; *** p<0.001 compared to indicated group.
**FIGURE 2****:** Growth retardation capacity improves after antiviral therapy in patients with chronic hepatitis C infection (n=140). Once the virus is reduced/eradicated a significant improvement in humoral immune response was observed in patients with no/mild fibrosis (Fib 0-1), advanced fibrosis (Fib 2-3) or cirrhosis (Fib 4). Significant differences between time points are indicated by horizontal black bars.

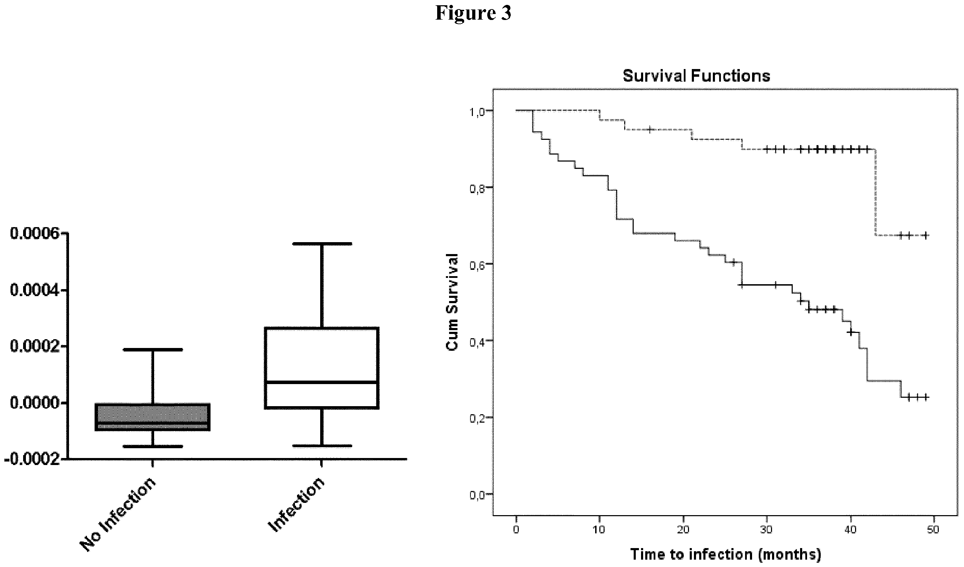

**FIGURE 3****:** Patients with cirrhosis (n=88) and impaired humoral immune responses are more susceptible to develop severe infections. Left panel: Growth retardation capacities of patients with liver cirrhosis who developed a severe infection within the observation period compared to patients with liver cirrhosis who did not develop a severe infection. Right panel: Survival function depicting the risk to develop a severe infection of patients with cirrhosis with intact (dashed line) or impaired (black line) growth retardation capacity. (Log-rank test. P<0.001).

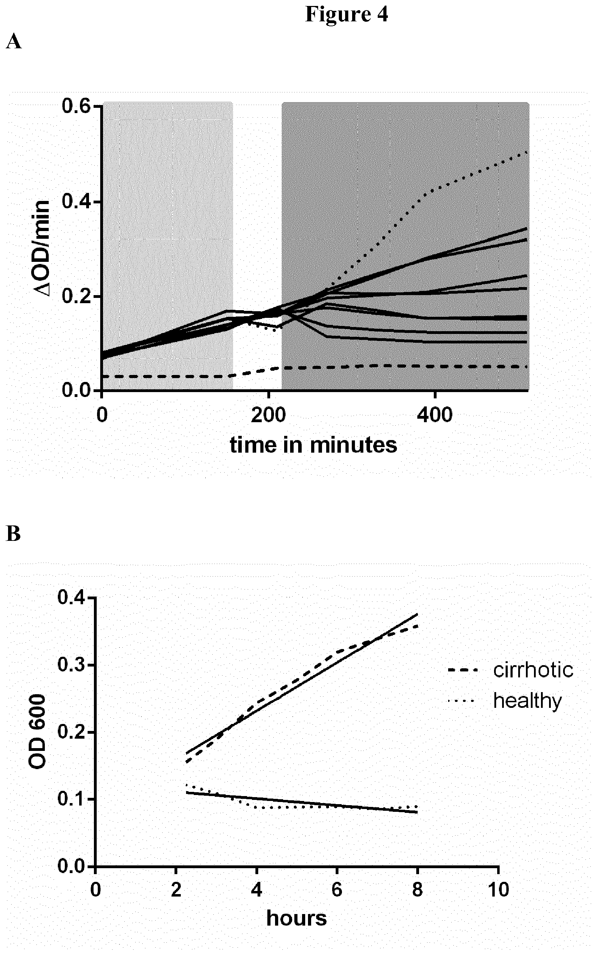

**FIGURE 4****:** Changes in optical density over time as measurement of bacterial growth in the presence and absence of serum. **(A)** shows a typical graph produced during the assay with bacteria growing to log phase without serum (light gray field), the incision in optical density to various degrees when serum is added after a period of unrestricted growth (white field) and inhibited or uninhibited growth of the bacteria when challenged with serum (dark gray field). The dotted line illustrates unrestricted growth of the bacteria and the addition of fresh medium instead of serum. The dashed line illustrates the minimal changes in optical density of sterile growth medium. The black lines are representative changes of bacterial growth in the presence of serum. **(B)** illustrates a possible way to analyse the readout. Data points after the addition of serum are used to fit a linear regression curve. It is obvious that the optical density in case of the cirrhotic serum (lipemic) is not as reduced as by the healthy sample.

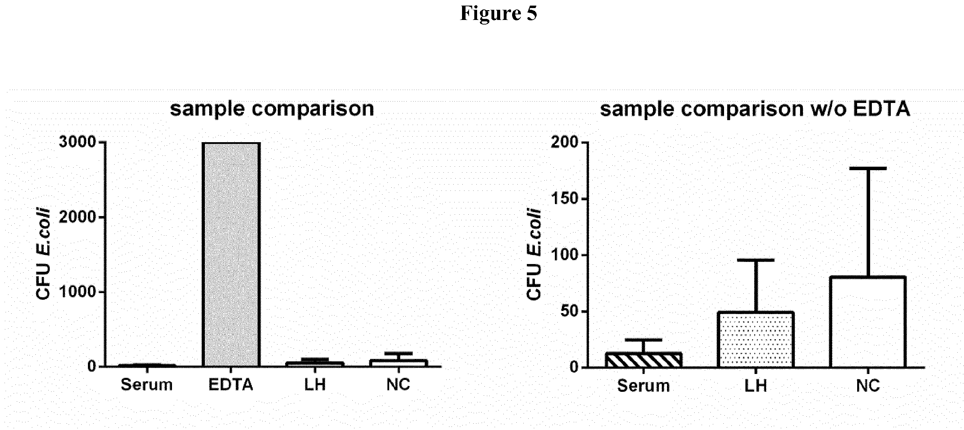

**FIGURE 5****:** Comparison of bacterial growth in the presence of serum and various types of plasma of four healthy controls. The left panel shows counted colony forming units after being challenged with serum, EDTA-plasma, lithium heparin (LH) plasma and sodium citrate (NC) obtained from the same four individuals. The right panel shows subtle differences between serum, LH-plasma and NC-plasma.

### EXAMPLES

The examples given below are for illustrative purposes only and do not limit the invention described above in any way.

### Materials & Methods

For the determination of the growth retardation capacity (measurement used to describe the state of the humoral immune system) of tested samples, *E. coli* XL1 blue bacteria were enumerated in an overnight culture (the *E. coli* XL1 blue bacteria may, for example, be ordered from Agilent (Santa Clara, USA, Catalog Number #200249)). For this purpose 5ml of sterile LB-broth (Sigma Aldrich, Saint Louis, USA) in a sterile 50ml reaction tube were inoculated with a scratch sample of a frozen glycerol stock taken with a sterile pipette tip. The reaction tube was loosely closed in a way that air flow was still possible. The reaction tube was then placed in a shaking incubator in a tilted position and incubated overnight (approximately 16 hours) at 37°C and 150rpm. The overnight culture was thoroughly mixed the next day and diluted 1:20 in fresh growth medium and again vortexed for at least 30 seconds. The bacterial suspension was transferred to a sterile, clear, flat bottom, 96-well plate. Each well was filled with 100µl of suspension except for two wells which were filled with fresh, sterile growth medium. Optical density of all wells was determined at a wavelength of 600nm. Then the plate was loosely covered and incubated at 37°C for 2.5 hours, sufficient to bring the bacteria in log phase. After 2.5 hours the plate was measured in the photometer again and the density was checked for sufficient bacterial growth. Immediately following the second measurement, 100µl of fresh growth medium or serum samples (thawed if necessary, thoroughly mixed and diluted 1:10 in fresh growth medium) were added to the bacterial suspension in a way that the following information could be retrieved at the end of the experiment.
1. The growth medium was in fact sterile. The designated wells without bacterial content were refreshed with growth medium and the optical density should stay virtually unchanged throughout the experiment.
2. The bacteria used for the experiment show in fact sufficient growth when uninhibited. Two wells with bacteria grown to log phase were refreshed with growth medium and the optical density should constantly increase throughout the experiment after an initial drop in optical density caused by the dilution with the freshly added medium.
3. To be tested, serum samples influence the growth of the bacteria in various possible ways. Prepared serum samples are added in duplicates to the bacteria grown to log phase and the optical density either increases, decreases or stays unchanged throughout the experiment. To account for density changes caused by different densities of serum samples (lipemic, icteric, hemolytic) the optical density obtained immediately after the mixture is used as an individual baseline value to which the following changes in optical density are compared.

As soon as possible after the mixture of bacterial suspension and test samples the optical density is measured and hourly after that for 5 hours.

To determine the growth retardation capacity of the tested samples, a linear regression line was fitted (least square difference) through all the available data points starting from the time point immediately after the mixture until the last time point. The slope of the resulting line was used to compare the growth retardation capacity of various samples. The smaller the values, the better the growth retardation capacity.

Growth retardation capacity was measured in various cohorts of liver disease patients and compared to healthy controls. All patients and volunteers gave written informed consent. Blood samples were drawn aseptically from a peripheral vein, using pyrogen-free tubes (VACUETTE®, Greiner Bio-One, Kremsmuenster, Austria), centrifuged for 10 minutes at 3000rpm (serum samples were kept on room temperature for 30 minutes until coagulation occurred), and frozen in small portions at -80°C until further use. Samples were collected from various already established cohorts at the Medical University of Graz (Department of Gastroenterology and Hepatology, Department of Transplantation Surgery). Cohort 1 (also referred to as PIC) comprised patients with cirrhosis (verified by a combination of typical clinical or radiological features or a liver biopsy) of various aetiologies including alcohol, chronic hepatitis B or C infection, NAFLD, hemochromatosis, Morbus Wilson, primary biliary cirrhosis, primary sclerosing cholangitis, and unclear reasons. The following exclusion criteria applied: Child-Pugh score 12 or higher, alcohol abuse within 2 weeks prior to inclusion, active infection at screening, antibiotic therapy except for permanent prophylaxis, simultaneous intake of pro-/pre-/symbiotic, gastrointestinal haemorrhage within 2 weeks prior to inclusion, immunomodulation drugs, hepatic encephalopathy stage two or higher, renal failure (creatinine over 1.7 mg/dL), pancreatitis, other severe diseases unrelated to cirrhosis, malignancy, suspected noncompliance, pregnancy, (clinicaltrials.gov: NCT01607528) Patients were clinically followed-up for an average of 37 months (1-50) to record events of severe infections, defined as infections in need of hospitalization.

Cohort 2 (also referred to as NEUTRO) comprised patients with chronic hepatitis C infection and various degrees of liver fibrosis/cirrhosis (categorized by transient elastography of the liver into no/mild fibrosis, advanced fibrosis and cirrhosis). These patients underwent therapy to eradicate the viral infection and were followed up to determine growth retardation capacity throughout their therapy/recovery. Blood samples were collected before the therapy (T0), immediately after the end of treatment (T2) and 12-24 weeks after the end of treatment (SVR). Patients with immunosuppressive medication, active infection at baseline or treatment with antibiotics within the preceding two weeks were excluded, (clinicaltrials.gov: NCT02545335).

Healthy controls had no sign of liver disease, susceptibility to develop infections, or any other chronic disease. The respective studies were approved by the institutional review board/ethics committee of the Medical University of Graz.

Statistical analysis was done with SPSS 23.0 (IBM Germany GmbH, Ehningen, Germany). Between-group differences were assessed by Mann-Whitney/Wilcoxon signed rank tests and Kruskal-Wallis/Friedman tests for unpaired/paired data to compare two or more groups respectively. Multiple comparisons with Bonferroni correction were used as post hoc tests. For survival analysis Kaplan-Meier curves were compared with Log Rank tests. Cutoffs were established using Youden-indices. All tests were performed on a 5% significance level.

### Results

Growth retardation capacity was severely impaired in patients with liver disease, in particular with liver cirrhosis. The most pronounced impairment was observed in patients with chronic hepatitis C infection, where the advancement of fibrosis from absent/mild to cirrhosis contributed significantly to the deterioration of the humoral immune system **(****FIGURE 1****).** Patients with chronic hepatitis C infection undergoing antiviral therapy showed a significant improvement in growth retardation capacity, regardless of their initial grade of fibrosis. **(****FIGURE 2****).**

Patients with various aetiologies of cirrhosis were clinically followed-up and severe infectious events were documented. Patients who developed a severe infection in the observation period had significantly worse growth retardation capacity at baseline compared to the patients that did not develop an infection. Growth retardation capacity values of -0.000045 (delta OD/min) or higher were predictive for the development of severe infections in patients with liver cirrhosis (p<0.001) **(****FIGURE 3****).**
Characteristics of a high quality experiment can be observed by continuous monitoring of optical density of the test mixtures. A linear increase in optical density before the addition of serum samples, an incision of optical density of the growth control, the continuous growth of bacteria in the growth control, and the absence of growth in the negative control should be visible in a successful experiment. For a reliable comparison of growth retardation capacities, a linear regression line should be fitted through a series of data points and the slope of the said line is a robust measurement for the comparison of growth retardation capacities between groups or between time points in repeated measurements **(****FIGURE 4****).**

For the data presented here, serum samples were used. However, the use of plasma (except for EDTA plasma) is also possible even though the samples with different additives are not directly comparable. As shown in **FIGURE 5****,** bacteria can grow uninhibited in EDTA plasma, and are killed to various degrees by serum, lithium heparin plasma and sodium citrate plasma. Growth retardation capacity can be measured from fresh or frozen samples, whereas repeated freeze and thaw cycles are tolerated.

## Claims

1. A method of determining the state of the humoral immune system in a patient comprising the steps of:
(i) providing a mixture consisting of a blood sample obtained from the patient and a bacteria suspension, wherein the bacteria are antimicrobial peptide (AMP) sensitive,
(ii) incubating the mixture, and
(iii) determining the change in density of the mixture over time.

2. The method of claim 1, wherein the change in density is compared to a change in reference density over time.

3. The method of claim 2, wherein
(i) the change in reference density is the change in density determined by measuring a reference mixture consisting of a blood sample obtained from a subject having an intact humoral immune system and a bacteria suspension,
(ii) the change in reference density is the change in density determined by measuring at least one reference sample of a mixture consisting of a blood sample obtained from a subject having an intact humoral immune system and a bacteria suspension,
(iii) the change in reference density is the change in density determined by measuring a reference bacteria suspension (not mixed with a blood sample), and/or
(iv) the change in reference density is the change in density determined by measuring at least one reference sample of a bacteria suspension (not mixed with a blood sample).

4. The method of any one of claims 1 to 3, wherein said determining comprises determining the density of the mixture at a first point in time and at least at one later point in time and comparing the densities determined at the different time points.

5. A method of determining the susceptibility of a patient to develop an infection comprising the steps of:
(i) providing a mixture consisting of a blood sample obtained from the patient and a bacteria suspension, wherein the bacteria are antimicrobial peptide (AMP) sensitive,
(ii) incubating the mixture, and
(iii) determining the change in density of the mixture over time.

6. The method of claim 5, wherein the change in density is compared to a change in reference density over time.

7. The method of claim 6, wherein
(i) the change in reference density is the change in density determined by measuring a reference mixture consisting of a blood sample obtained from a subject having no susceptibility to develop an infection (= having an intact humoral immune system) and a bacteria suspension,
(ii) the change in reference density is the change in density determined by measuring at least one reference sample of a mixture consisting of a blood sample obtained from a subject having no susceptibility to develop an infection (= having an intact humoral immune system) and a bacteria suspension,
(iii) the change in reference density is the change in density determined by measuring a reference bacteria suspension (not mixed with a blood sample), and/or
(iv) the change in reference density is the change in density determined by measuring at least one reference sample of a bacteria suspension (not mixed with a blood sample).

8. The method of any one of claims 5 to 7, wherein said determining comprises determining the density of the mixture at a first point in time and at least at one later point in time and comparing the densities determined at the different time points.

9. A method of monitoring the state of the humoral immune system in a patient comprising the steps of:
(a) carrying out an incubation cycle comprising
(i) providing a mixture consisting of a blood sample obtained from the patient and a bacteria suspension, wherein the bacteria are antimicrobial peptide (AMP) sensitive,
(ii) incubating the mixture, and
(iii) determining the change in density of the mixture over time, wherein said determining comprises determining the density of the mixture at least at one first point in time and at least at one second point in time and comparing the densities determined at the different time points, and
(b) carrying out at least one further incubation cycle comprising
(i) providing a mixture consisting of a blood sample obtained from the (same) patient and a bacteria suspension, wherein the bacteria are antimicrobial peptide (AMP) sensitive,
(ii) incubating the mixture, and
(iii) determining the change in density of the mixture over time, wherein said determining comprises determining the density of the mixture at least at one third point in time and at least at one fourth point in time and comparing the densities determined at the different time points.

10. A method of monitoring the susceptibility of a patient to develop an infection comprising the steps of:
(a) carrying out an incubation cycle comprising
(i) providing a mixture consisting of a blood sample obtained from the patient and a bacteria suspension, wherein the bacteria are antimicrobial peptide (AMP) sensitive,
(ii) incubating the mixture, and
(iii) determining the change in density of the mixture over time, wherein said determining comprises determining the density of the mixture at least at one first point in time and at least at one second point in time and comparing the densities determined at the different time points, and
(b) carrying out at least one further incubation cycle comprising
(i) providing a mixture consisting of a blood sample obtained from the (same) patient and a bacteria suspension, wherein the bacteria are antimicrobial peptide (AMP) sensitive,
(ii) incubating the mixture, and
(iii) determining the change in density of the mixture over time, wherein said determining comprises determining the density of the mixture at least at one third point in time and at least at one fourth point in time and comparing the densities determined at the different time points.

11. Use of a bacteria suspension to determine the state of the humoral immune system in a blood sample obtained from a patient, wherein the bacteria suspension and the blood sample form a mixture consisting of the bacteria suspension and the blood sample, and wherein the bacteria are antimicrobial peptide (AMP) sensitive.

12. Use of a bacteria suspension to determine the susceptibility of a patient to develop an infection in a blood sample obtained from the patient, wherein the bacteria suspension and the blood sample form a mixture consisting of the bacteria suspension and the blood sample, and wherein the bacteria are antimicrobial peptide (AMP) sensitive.

13. Use of a bacteria suspension to monitor the state of the humoral immune system in a blood sample obtained from a patient, wherein the bacteria suspension and the blood sample form a mixture consisting of the bacteria suspension and the blood sample, and wherein the bacteria are antimicrobial peptide (AMP) sensitive.

14. Use of a bacteria suspension to monitor the susceptibility of a patient to develop an infection in a blood sample obtained from the patient, wherein the bacteria suspension and the blood sample form a mixture consisting of the bacteria suspension and the blood sample, and wherein the bacteria are antimicrobial peptide (AMP) sensitive.

15. Use of a kit for determining the state of the humoral immune system in a patient, the susceptibility of a patient to develop an infection, monitoring the state of the humoral immune system in a patient, and/or monitoring the susceptibility of a patient to develop an infection consisting of:
(i) antimicrobial peptide (AMP) sensitive bacteria,
(ii) means for sampling blood from a patient, and/or
(iii) means for preparing a suspension of said bacteria and/or cultivating the suspension of said bacteria and/or a mixture consisting of the suspension of said bacteria and the blood sample.

## Patentansprüche

1. Verfahren zum Bestimmen des Zustands des humoralen Immunsystems bei einem Patienten, umfassend die folgenden Schritte:
(i) Bereitstellen eines Gemischs, bestehend aus einer von dem Patienten erlangten Blutprobe und einer Bakteriensuspension, wobei die Bakterien sensitiv auf antimikrobielles Peptid (AMP) sind,
(ii) Inkubieren des Gemischs, und
(iii) Bestimmen der Dichteänderung des Gemischs über die Zeit.

2. Verfahren nach Anspruch 1, wobei die Dichteänderung mit einer Änderung einer Referenzdichte über die Zeit verglichen wird.

3. Verfahren nach Anspruch 2, wobei
(i) die Änderung einer Referenzdichte die Dichteänderung ist, die durch Messen eines Referenzgemischs bestimmt wird, das aus einer Blutprobe, die von einem Subjekt erlangt wurde, das ein intaktes humorales Immunsystem aufweist, und einer Bakteriensuspension besteht,
(ii) die Änderung einer Referenzdichte die Dichteänderung ist, die durch Messen mindestens einer Referenzprobe eines Gemischs bestimmt wird, das aus einer Blutprobe, die von einem Subjekt erlangt wurde, das ein intaktes humorales Immunsystem aufweist, und einer Bakteriensuspension besteht,
(iii) die Änderung einer Referenzdichte die Dichteänderung ist, die durch Messen einer Referenzbakteriensuspension (nicht gemischt mit einer Blutprobe) bestimmt wird, und/oder
(iv) die Änderung der Referenzdichte die Dichteänderung ist, die durch Messen mindestens einer Referenzprobe einer Bakteriensuspension (nicht gemischt mit einer Blutprobe) bestimmt wird.

4. Verfahren nach einem der Ansprüche 1 bis 3, wobei das Bestimmen ein Bestimmen der Dichte des Gemischs zu einem ersten Zeitpunkt und zu mindestens einem späteren Zeitpunkt und ein Vergleichen der zu den verschiedenen Zeitpunkten bestimmten Dichten umfasst.

5. Verfahren zum Bestimmen der Anfälligkeit eines Patienten, eine Infektion zu entwickeln, umfassend die folgenden Schritte:
(i) Bereitstellen eines Gemischs, bestehend aus einer von dem Patienten erlangten Blutprobe und einer Bakteriensuspension, wobei die Bakterien sensitiv auf antimikrobielles Peptid (AMP) sind,
(ii) Inkubieren des Gemischs, und
(iii) Bestimmen der Dichteänderung des Gemischs über die Zeit.

6. Verfahren nach Anspruch 5, wobei die Dichteänderung mit einer Änderung einer Referenzdichte über die Zeit verglichen wird.

7. Verfahren nach Anspruch 6, wobei
(i) die Änderung einer Referenzdichte die Dichteänderung ist, die durch Messen eines Referenzgemischs bestimmt wird, das aus einer Blutprobe, die von einem Subjekt erlangt wurde, das keine Anfälligkeit aufweist, eine Infektion zu entwickeln (= das ein intaktes humorales Immunsystem aufweist), und einer Bakteriensuspension besteht,
(ii) die Änderung einer Referenzdichte die Dichteänderung ist, die durch Messen mindestens einer Referenzprobe eines Gemischs bestimmt wird, das aus einer Blutprobe, die von einem Subjekt erlangt wurde, das keine Anfälligkeit aufweist, eine Infektion zu entwickeln (= das ein intaktes humorales Immunsystem aufweist), und einer Bakteriensuspension besteht,
(iii) die Änderung einer Referenzdichte die Dichteänderung ist, die durch Messen einer Referenzbakteriensuspension (nicht gemischt mit einer Blutprobe) bestimmt wird, und/oder
(iv) die Änderung der Referenzdichte die Dichteänderung ist, die durch Messen mindestens einer Referenzprobe einer Bakteriensuspension (nicht gemischt mit einer Blutprobe) bestimmt wird.

8. Verfahren nach einem der Ansprüche 5 bis 7, wobei das Bestimmen ein Bestimmen der Dichte des Gemischs zu einem ersten Zeitpunkt und zu mindestens einem späteren Zeitpunkt und ein Vergleichen der zu den verschiedenen Zeitpunkten bestimmten Dichten umfasst.

9. Verfahren zum Überwachen des Zustands des humoralen Immunsystems bei einem Patienten, umfassend die folgenden Schritte:
(a) Durchführen eines Inkubationszyklus, umfassend
(i) Bereitstellen eines Gemischs, bestehend aus einer von dem Patienten erlangten Blutprobe und einer Bakteriensuspension, wobei die Bakterien sensitiv auf antimikrobielles Peptid (AMP) sind,
(ii) Inkubieren des Gemischs, und
(iii) Bestimmen der Dichteänderung des Gemischs über die Zeit, wobei das Bestimmen ein Bestimmen der Dichte des Gemischs mindestens zu einem ersten Zeitpunkt und zu mindestens einem zweiten Zeitpunkt und ein Vergleichen der zu den verschiedenen Zeitpunkten bestimmten Dichten umfasst, und
(b) Durchführen mindestens eines weiteren Inkubationszyklus, umfassend
(i) Bereitstellen eines Gemischs, bestehend aus einer von dem (gleichen) Patienten erlangten Blutprobe und einer Bakteriensuspension, wobei die Bakterien sensitiv auf antimikrobielles Peptid (AMP) sind,
(ii) Inkubieren des Gemischs, und
(iii) Bestimmen der Dichteänderung des Gemischs über die Zeit, wobei das Bestimmen ein Bestimmen der Dichte des Gemischs mindestens zu einem dritten Zeitpunkt und zu mindestens einem vierten Zeitpunkt und ein Vergleichen der zu den verschiedenen Zeitpunkten bestimmten Dichten umfasst.

10. Verfahren zum Überwachen der Anfälligkeit eines Patienten, eine Infektion zu entwickeln, umfassend die folgenden Schritte:
(a) Durchführen eines Inkubationszyklus, umfassend
(i) Bereitstellen eines Gemischs, bestehend aus einer von dem Patienten erlangten Blutprobe und einer Bakteriensuspension, wobei die Bakterien sensitiv auf antimikrobielles Peptid (AMP) sind,
(ii) Inkubieren des Gemischs, und
(iii) Bestimmen der Dichteänderung des Gemischs über die Zeit, wobei das Bestimmen ein Bestimmen der Dichte des Gemischs mindestens zu einem ersten Zeitpunkt und zu mindestens einem zweiten Zeitpunkt und ein Vergleichen der zu den verschiedenen Zeitpunkten bestimmten Dichten umfasst, und
(b) Durchführen mindestens eines weiteren Inkubationszyklus, umfassend
(i) Bereitstellen eines Gemischs, bestehend aus einer von dem (gleichen) Patienten erlangten Blutprobe und einer Bakteriensuspension, wobei die Bakterien sensitiv auf antimikrobielles Peptid (AMP) sind,
(ii) Inkubieren des Gemischs, und
(iii) Bestimmen der Dichteänderung des Gemischs über die Zeit, wobei das Bestimmen ein Bestimmen der Dichte des Gemischs mindestens zu einem dritten Zeitpunkt und zu mindestens einem vierten Zeitpunkt und ein Vergleichen der zu den verschiedenen Zeitpunkten bestimmten Dichten umfasst.

11. Verwendung einer Bakteriensuspension zum Bestimmen des Zustands des humoralen Immunsystems in einer Blutprobe, die von einem Patienten erlangt wird, wobei die Bakteriensuspension und die Blutprobe ein Gemisch bilden, das aus der Bakteriensuspension und der Blutprobe besteht, und wobei die Bakterien sensitiv auf antimikrobielles Peptid (AMP) sind.

12. Verwendung einer Bakteriensuspension zum Bestimmen der Anfälligkeit eines Patienten, eine Infektion zu entwickeln, in einer von dem Patienten erlangten Blutprobe, wobei die Bakteriensuspension und die Blutprobe ein Gemisch bilden, das aus der Bakteriensuspension und der Blutprobe besteht, und wobei die Bakterien sensitiv auf antimikrobielles Peptid (AMP) sind.

13. Verwendung einer Bakteriensuspension zum Überwachen des Zustands des humoralen Immunsystems in einer Blutprobe, die von einem Patienten erlangt wird, wobei die Bakteriensuspension und die Blutprobe ein Gemisch bilden, das aus der Bakteriensuspension und der Blutprobe besteht, und wobei die Bakterien sensitiv auf antimikrobielles Peptid (AMP) sind.

14. Verwendung einer Bakteriensuspension zum Überwachen der Anfälligkeit eines Patienten, eine Infektion zu entwickeln, in einer von dem Patienten erlangten Blutprobe, wobei die Bakteriensuspension und die Blutprobe ein Gemisch bilden, das aus der Bakteriensuspension und der Blutprobe besteht, und wobei die Bakterien sensitiv auf antimikrobielles Peptid (AMP) sind.

15. Verwendung eines Kits zum Bestimmen des Zustands des humoralen Immunsystems bei einem Patienten, zum Bestimmen der Anfälligkeit eines Patienten, eine Infektion zu entwickeln, zum Überwachen des Zustands des humoralen Immunsystems bei einem Patienten und/oder zum Überwachen der Anfälligkeit eines Patienten, eine Infektion zu entwickeln, bestehend aus:
(i) auf antimikrobielles Peptid (AMP) sensitive Bakterien,
(ii) Mittel zur Entnahme von Blut bei einem Patienten, und/oder
(iii) Mittel zum Herstellen einer Suspension der Bakterien und/oder zum Kultivieren der Suspension der Bakterien und/oder eines Gemischs, das aus der Suspension der Bakterien und der Blutprobe besteht.

## Revendications

1. Procédé permettant de déterminer l'état du système immunitaire humoral chez un patient comprenant les étapes de :
(i) fourniture d'un mélange constitué d'un échantillon de sang prélevé chez le patient et d'une suspension bactérienne, les bactéries étant sensibles aux peptides antimicrobiens (AMP),
(ii) incubation du mélange, et
(iii) détermination du changement de densité du mélange au cours du temps.

2. Procédé selon la revendication 1, ledit changement de densité étant comparé à un changement de densité de référence au cours du temps.

3. Procédé selon la revendication 2,
(i) ledit changement de densité de référence étant le changement de densité déterminé en mesurant un mélange de référence constitué d'un échantillon de sang prélevé chez un sujet doté d'un système immunitaire humoral intact et d'une suspension bactérienne,
(ii) ledit changement de densité de référence étant le changement de densité déterminé en mesurant au moins un échantillon de référence d'un mélange constitué d'un échantillon de sang prélevé chez un sujet doté d'un système immunitaire humoral intact et d'une suspension bactérienne,
(iii) ledit changement de densité de référence étant le changement de densité déterminé en mesurant une suspension bactérienne de référence (non mélangée à un échantillon de sang), et/ou
(iv) ledit changement de densité de référence étant le changement de densité déterminé en mesurant au moins un échantillon de référence d'une suspension bactérienne (non mélangée à un échantillon de sang).

4. Procédé selon l'une quelconque des revendications 1 à 3, ladite détermination comprenant la détermination de la densité du mélange à un premier moment dans le temps et au moins à un moment ultérieur dans le temps et la comparaison des densités déterminées aux différents moments dans le temps.

5. Procédé permettant de déterminer la susceptibilité d'un patient à développer une infection comprenant les étapes de :
(i) fourniture d'un mélange constitué d'un échantillon de sang prélevé chez le patient et d'une suspension bactérienne, les bactéries étant sensibles aux peptides antimicrobiens (AMP),
(ii) incubation du mélange, et
(iii) détermination du changement de densité du mélange au cours du temps.

6. Procédé selon la revendication 5, ledit changement de densité étant comparé à un changement de densité de référence au cours du temps.

7. Procédé selon la revendication 6,
(i) ledit changement de densité de référence étant le changement de densité déterminé en mesurant un mélange de référence constitué d'un échantillon de sang prélevé chez un sujet ne présentant aucune susceptibilité à développer une infection (= doté d'un système immunitaire humoral intact) et d'une suspension bactérienne,
(ii) ledit changement de densité de référence étant le changement de densité déterminé en mesurant au moins un échantillon de référence d'un mélange constitué d'un échantillon de sang prélevé chez un sujet ne présentant aucune susceptibilité à développer une infection (= doté d'un système immunitaire humoral intact) et d'une suspension bactérienne,
(iii) ledit changement de densité de référence étant le changement de densité déterminé en mesurant une suspension bactérienne de référence (non mélangée à un échantillon de sang), et/ou
(iv) ledit changement de densité de référence étant le changement de densité déterminé en mesurant au moins un échantillon de référence d'une suspension bactérienne (non mélangée à un échantillon de sang).

8. Procédé selon l'une quelconque des revendications 5 à 7, ladite détermination comprenant la détermination de la densité du mélange à un premier moment dans le temps et au moins à un moment ultérieur dans le temps et la comparaison des densités déterminées aux différents moments dans le temps.

9. Procédé de surveillance de l'état du système immunitaire humoral chez un patient comprenant les étapes de :
(a) réalisation d'un cycle d'incubation comprenant
(i) la fourniture d'un mélange constitué d'un échantillon de sang prélevé chez le patient et d'une suspension bactérienne, les bactéries étant sensibles aux peptides antimicrobiens (AMP),
(ii) l'incubation du mélange, et
(iii) la détermination du changement de densité du mélange au cours du temps, ladite détermination comprenant la détermination de la densité du mélange au moins à un premier moment dans le temps et au moins à un deuxième moment dans le temps et la comparaison des densités déterminées aux différents moments dans le temps, et
(b) réalisation d'au moins un autre cycle d'incubation comprenant
(i) la fourniture d'un mélange constitué d'un échantillon de sang prélevé chez le (même) patient et d'une suspension bactérienne, les bactéries étant sensibles aux peptides antimicrobiens (AMP),
(ii) l'incubation du mélange, et
(iii) la détermination du changement de densité du mélange au cours du temps, ladite détermination comprenant la détermination de la densité du mélange au moins à un troisième moment dans le temps et au moins à un quatrième moment dans le temps et la comparaison des densités déterminées aux différents moments dans le temps.

10. Procédé de surveillance de la susceptibilité d'un patient à développer une infection comprenant les étapes de :
(a) réalisation d'un cycle d'incubation comprenant
(i) la fourniture d'un mélange constitué d'un échantillon de sang prélevé chez le patient et d'une suspension bactérienne, les bactéries étant sensibles aux peptides antimicrobiens (AMP),
(ii) l'incubation du mélange, et
(iii) la détermination du changement de densité du mélange au cours du temps, ladite détermination comprenant la détermination de la densité du mélange au moins à un premier moment dans le temps et au moins à un deuxième moment dans le temps et la comparaison des densités déterminées aux différents moments dans le temps, et
(b) réalisation d'au moins un autre cycle d'incubation comprenant
(i) la fourniture d'un mélange constitué d'un échantillon de sang prélevé chez le (même) patient et d'une suspension bactérienne, les bactéries étant sensibles aux peptides antimicrobiens (AMP),
(ii) l'incubation du mélange, et
(iii) la détermination du changement de densité du mélange au cours du temps, ladite détermination comprenant la détermination de la densité du mélange au moins à un troisième moment dans le temps et au moins à un quatrième moment dans le temps et la comparaison des densités déterminées aux différents moments dans le temps.

11. Utilisation d'une suspension bactérienne pour déterminer l'état du système immunitaire humoral dans un échantillon de sang prélevé chez un patient, ladite suspension bactérienne et ledit échantillon de sang formant un mélange constitué de la suspension bactérienne et de l'échantillon de sang, et lesdites bactéries étant sensibles aux peptides antimicrobiens (AMP).

12. Utilisation d'une suspension bactérienne pour déterminer la susceptibilité d'un patient à développer une infection dans un échantillon de sang prélevé chez le patient, ladite suspension bactérienne et ledit échantillon de sang formant un mélange constitué de la suspension bactérienne et de l'échantillon de sang, et lesdites bactéries étant sensibles aux peptides antimicrobiens (AMP).

13. Utilisation d'une suspension bactérienne pour surveiller l'état du système immunitaire humoral dans un échantillon de sang prélevé chez un patient, ladite suspension bactérienne et ledit échantillon de sang formant un mélange constitué de la suspension bactérienne et de l'échantillon de sang, et lesdites bactéries étant sensible aux peptides antimicrobiens (AMP).

14. Utilisation d'une suspension bactérienne pour surveiller la susceptibilité d'un patient à développer une infection dans un échantillon de sang prélevé chez le patient, ladite suspension bactérienne et ledit échantillon de sang formant un mélange constitué de la suspension bactérienne et de l'échantillon de sang, et lesdites bactéries étant sensibles aux peptides antimicrobiens (AMP).

15. Utilisation d'un kit permettant de déterminer l'état du système immunitaire humoral chez un patient, la susceptibilité d'un patient à développer une infection, de surveiller l'état du système immunitaire humoral chez un patient et/ou de surveiller la susceptibilité d'un patient à développer une infection, constitué de :
(i) bactéries sensibles aux peptides antimicrobiens (AMP),
(ii) moyens pour prélever du sang chez un patient, et/ou
(iii) moyens pour préparer une suspension desdites bactéries et/ou cultiver la suspension desdites bactéries et/ou un mélange constitué de la suspension desdites bactéries et de l'échantillon de sang.
